# EUROPEAN PATENT APPLICATION

(11) **EP 1 475 388 A2**
(43) Date of publication of application: **10.11.2004**
(21) Application number: 04075961.5
(22) Date of filing: 18.07.2000
(51) Int. Cl.: C07K 14/47, C07K 16/18, G01N 33/53, G01N 33/567, C12N 5/10, C12N 15/12, C12N 15/63, C12N 15/64, C07K 14/705

(54) **Novel human calcium sensitive potassium channel subunits**

(30) Priority: 20.07.1999 US 144764 P
(62) Divisional of application: 00950422.6
(71) Applicant: Merck & Co., Inc., Rahway New Jersey 07065-0900 (US)
(72) Inventor: Uebele, Victor, Rahway, NJ 07065-0907 (US); Swanson, Richard, Rahway, NJ 07065-0907 (US); Liu, Yuan, Rahway, NJ 07065-0907 (US); Lagrutta, Armando, Rahway, NJ 07065-0907 (US)
(74) Representative: Horgan, James Michael Frederic

(57) **Abstract**

The present invention is directed to novel human DNA sequences encoding calcium sensitive potassium channel subunits β3a, β3b, β3c and β3d, the proteins encoded by the DNA sequences, vectors comprising the DNA sequences, host cells containing the vectors, and methods of identifying inhibitors and agonists of calcium sensitive potassium channels containing human β3a, β3b, β3c, or β3d subunits and inhibitors and agonists of β3 gene transcription.

## Description

### CROSS-REFERENCE TO RELATED APPLICATIONS

Not applicable.

### STATEMENT REGARDING FEDERALLY-SPONSORED R&D

Not applicable.

### REFERENCE TO MICROFICHE APPENDIX

Not applicable.

### FIELD OF THE INVENTION

The present invention is directed to novel human DNA sequences encoding subunits of calcium sensitive potassium channels.

### BACKGROUND OF THE INVENTION

Voltage-gated potassium channels form transmembrane pores that open or close in response to changes in cell membrane potential and selectively allow potassium ions to pass through the membrane. Voltage-gated potassium channels have been found in cells traditionally considered both excitable (*e.g*., neurons, myocytes, secretory cells) and non-excitable (*e.g*., T-cells, osteoclasts) and have been shown to maintain cell membrane potential and control the repolarization of action potentials in such cells. Following depolarization, voltage-gated potassium channels open, allowing potassium efflux and thus membrane repolarization. This behavior has made voltage-gated potassium channels important targets for drug discovery in connection with a variety of diseases. As a result, many voltage-gated potassium channels have been identified and many cloned. They are distinguishable by differences in primary structure and tissue-specific patterns of expression, as well as by electrophysiological and pharmacological properties. For reviews of voltage-gated potassium channels see Robertson, 1997, Trends Pharmacol. Sci. 18:474-483; Jan & Jan, 1997, J. Physiol. 505:267-282; Catterall, 1995, Ann. Rev. Biochem. 64:493-531.

Many functional voltage-gated potassium channels are believed to be tetramers of four α subunits, each of which contains six transmembrane spanning segments. The αsubunits making up a tetramer may be the same (in the case of homotetramers) or may be different (in the case of heterotetramers). The membrane-spanning α subunits making up the tetramers may sometimes be associated with additional, β subunits, which may alter the behavior of the α subunits.

A particular type of voltage-gated potassium channel is the voltage-gated and calcium sensitive potassium channel, also known as the calcium sensitive potassium channel. Calcium sensitive potassium channels are present in a wide variety of cells and are unique among voltage-gated potassium channels because their activity is regulated not only by changes in membrane potential but also by intracellular calcium concentration. Plasma membrane depolarization and increases in cytoplasmic calcium concentration both raise the open probability of calcium sensitive potassium channels. Therefore, calcium sensitive potassium channels can serve as a link between cellular processes involving increases in intracellular calcium and membrane excitability. Calcium sensitive potassium channels are believed to play a negative feedback role by terminating signaling events involving an increase in intracellular calcium, *e.g.,* glucose mediated insulin release, blood vessel muscle tone, bronchial airway smooth muscle tone, and regulation of intraocular pressure. (Tanaka et al., 1997, J. Physiol. 502:545-557; Kaczorowski et al., 1996, J. Bioenerg. Biomem. 28:255-267; Vergara et al., 1998, Curr. Opin. Neurobiol. 8:321-329).

Certain calcium sensitive potassium channels have been isolated and studied. Functional calcium sensitive potassium channels are composed of α subunits that may be associated with smaller β subunits. The α subunit is believed to form the channel pore while a previously described β subunit increases the calcium sensitivity of the channel and makes the channel susceptible to regulation by certain substances, *e.g*., dehydrosoyasaponin (McManus et al., 1995, Neuron 14:645-650). The calcium sensitive potassium channel from bovine tracheal smooth muscle was purified and shown to be composed of an ~ 130 kDa α subunit and a 31 kDa β subunit (Garcia-Calvo et al., 1994, J. Biol. Chem. 269:676-682). Tseng-Crank et al. (1994, Neuron 13:1315-1330) cloned nine related calcium sensitive potassium channel α subunits from human brain. These α subunits are thought to be splice variants derived from a single gene, the *h-slo* gene (Tseng-Crank et al., 1994, Neuron 13:1315-1330). Knauss et al., 1994, J. Biol. Chem. 269:17274-17278 purified and cloned a β subunit of a calcium sensitive potassium channel from tracheal smooth muscle.

In most cells, the opening of calcium sensitive potassium channels results in the generation of non-inactivating, hyperpolarizing potassium currents. However, in certain cells (*e.g*., chromaffin cells of the adrenal gland and hippocampal neurons), the currents are inactivating. Following the discovery of the invention described herein, Wallner et al., 1999, Proc. Natl. Acad. Sci. USA 96:4137-4132 disclosed the existence of the human β2 calcium sensitive potassium channel subunit that, when combined with the α subunit, formed inactivating calcium sensitive potassium channels. The ability to confer inactivation was ascribed to the N-terminal 19 amino acids of the β2 subunit.

U.S. Patent No. 5,776,734 is directed to nucleic acids encoding the bovine and human β1 subunit of the calcium sensitive potassium channel. U.S. Patent No. 5,637,470 is directed to methods of identifying compounds that modulate the activity of calcium sensitive potassium channels.

### SUMMARY OF THE INVENTION

The present invention is directed to novel human DNA sequences encoding β subunits of calcium sensitive potassium channels. The present invention includes DNAs that encode the β subunits β2, β3a, β3b, β3c, and β3d of human calcium sensitive potassium channels. The DNAs comprise the nucleotide sequences shown in SEQ.ID.NO.:1 (β2), SEQ.ID.NO.:3 (β3a), SEQ.ID.NO.:5 (β3b), SEQ.ID.NO.:7 (β3c), and SEQ.ID.NO.:9 (β3d). Also provided are proteins encoded by the novel DNA sequences. The proteins comprise the deduced amino acid sequences shown in SEQ.ID.NO.:2 (β2), SEQ.ID.NO.:4 (β3a), SEQ.ID.NO.:6 (β3b), SEQ.ID.NO.:8 (β3c), and SEQ.ID.NO.: 10 (β3d). Methods of expressing the novel subunit proteins in recombinant systems are provided as well as methods of identifying activators and inhibitors of potassium channels comprising the subunits.

The present invention also includes a genomic DNA fragment containing the 5' portions of the β3a, β3b, β3c, and β3d subunits, as well as the 5' portion of the core portion of the β3 subunits. This genomic DNA fragment contains promoter elements for the subunits. Methods of screening for compounds which affect transcription of the gene encoding the β3a, β3b, β3c, and β3d subunits are also provided.

### BRIEF DESCRIPTION OF THE DRAWINGS

Figure 1A shows a DNA sequence encoding the β2 subunit of the human calcium sensitive potassium channel (SEQ.ID.NO.:1). The start ATG codon is at position 271-273; the stop codon is at position 976-978. Figure 1B shows the deduced amino acid sequence (SEQ.ID.NO.:2) of the β2 subunit.
Figure 2A shows a DNA sequence encoding the β3a subunit of a human calcium sensitive potassium channel (SEQ.ID.NO.:3). The start ATG codon is at position 341-343; the stop codon is at position 1172-1174. Figure 2B shows the deduced amino acid sequence (SEQ.ID.NO.:4) of the β3a subunit.
Figure 3A shows a DNA sequence encoding the β3b subunit of a human calcium sensitive potassium channel (SEQ.ID.NO.:5). The start ATG codon is at position 796-798; the stop codon is at position 1567-1569. Figure 3B shows the deduced amino acid sequence (SEQ.ID.NO.:6) of the β3b subunit.
Figure 4A shows a DNA sequence encoding the β3c subunit of a human calcium sensitive potassium channel (SEQ.ID.NO.:7). The start ATG codon is at position 869-871; the stop codon is at position 1694-1696. Figure 4B shows the deduced amino acid sequence (SEQ.ID.NO.:8) of the β3c subunit.
Figure 5A shows a DNA sequence encoding the β3d subunit of a human and calcium sensitive potassium channel (SEQ.ID.NO.:9). The start ATG codon is at position 457-459; the stop codon is at position 1294-1296. Figure 5B shows the deduced amino acid sequence (SEQ.ID.NO.:10) of the β3d subunit.
Figure 6 shows an alignment of the deduced amino acid sequences of the human calcium sensitive potassium channel β1 (SEQ.ID.NO.:11), β2 (SEQ.ID.NO.:2), β3a (SEQ.ID.NO.:4), β3b (SEQ.ID.NO.:6), β3c (SEQ-ID.NO.:8), and β3d (SEQ.ID.NO.:10) subunits.

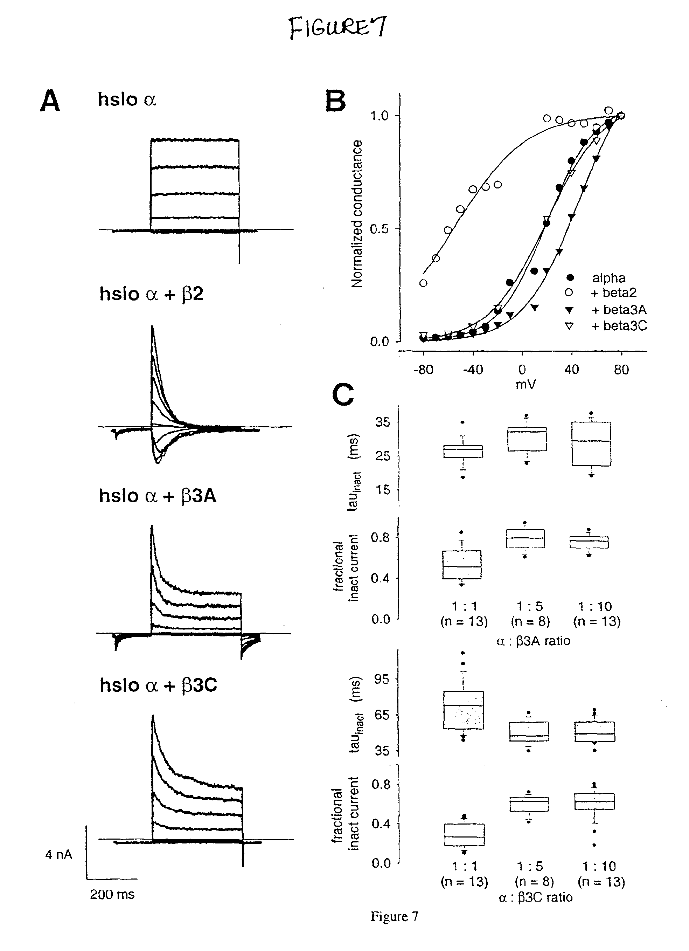

Figure 7 shows the effect of the co-expression of the novel β subunits of the present invention on the electrophysiological properties of the ion channel formed by the α subunit of a human calcium sensitive potassium channel. Figure 7A shows the current-voltage relations recorded in inside-out patches expressing calcium sensitive potassium channel α or α and β subunits. α and β subunit cRNAs were coinjected in 1:10 molar ratio (β in excess) to detect maximum effects. The voltage clamp protocol consisted of a pre-pulse to -160 mV (200 ms), followed by 20 mV depolarizing steps from -80 to +80 mV (500 ms); holding potential was -80 mV; internal Ca²⁺ was 30 µM. Subunits β3b and β3d did not induce noticeable changes in the kinetics and voltage dependence of the channels formed by α subunits, although they might decrease current density. Figure 7B: Boltzmann equations were fit to normalized conductances for the records shown in 7A, which were calculated from peak currents and plotted as function of test potential. V_{1/2} values are: 20 mV (α subunit alone); -55 mV (α +β2 subunit); 45.36 mV (α +β3a subunit); 20 mV (α + β3c subunit). Figure 7C shows that co-expression of β3 subunit RNAs in molar excess of α subunit RNAs (up to 10X) reduced, but did not eliminate, a non-inactivating component of calcium sensitive potassium channel current. Inactivation rates and fractional inactivating current were calculated as described in Example 2.
Figure 8A-N shows the genomic sequence of GenBank accession number AC007823.4 (SEQ.ID.NO.:20). The different splice variants of the β3 subunits are contained in nucleotides 1-40,467. The β3a-specific sequence is at positions 17,404-17,806; the β3b-specific sequence is at positions 24,710-25,507; the β3c/d sequence is at positions 32,590-33,514; the beginning of the β3 core sequence is at positions 33,515-33,705. The sequences involved in tissue specific expression (*e.g*., promoters, enhancers, repressors) are likely to be located in nucleotides 1-17,404.

### DETAILED DESCRIPTION OF THE INVENTION

For the purposes of this invention:

"Substantially free from other proteins" means at least 90%, preferably 95%, more preferably 99%, and even more preferably 99.9%, free of other proteins. Thus, a human calcium sensitive potassium channel β2, β3a, β3b, β3c, or β3d subunit protein preparation that is substantially free from other proteins will contain, as a percent of its total protein, no more than 10%, preferably no more than 5%, more preferably no more than 1%, and even more preferably no more than 0.1%, of non-human calcium sensitive potassium channel β2, β3a, β3b, β3c, or β3d subunit proteins. Whether a given human calcium sensitive potassium channel β2, β3a, β3b, β3c, or β3d subunit protein preparation is substantially free from other proteins can be determined by conventional techniques of assessing protein purity such as, *e.g*., sodium dodecyl sulfate polyacrylamide gel electrophoresis (SDS-PAGE) combined with appropriate detection methods, *e.g*., silver staining or immunoblotting.

"Substantially free from other nucleic acids" means at least 90%, preferably 95%, more preferably 99%, and even more preferably 99.9%, free of other nucleic acids. Thus, a human calcium sensitive potassium channel β2, β3a, β3b, β3c, or β3d subunit DNA preparation that is substantially free from other nucleic acids will contain, as a percent of its total nucleic acid, no more than 10%, preferably no more than 5%, more preferably no more than 1%, and even more preferably no more than 0.1%, of non-human calcium sensitive potassium channel β2, β3a, β3b, β3c, or β3d subunit nucleic acids. Whether a given human calcium sensitive potassium channel β2, β3a, β3b, β3c, or β3d subunit DNA preparation is substantially free from other nucleic acids can be determined by conventional techniques of assessing nucleic acid purity such as, *e.g*., agarose gel electrophoresis combined with appropriate staining methods, *e.g*., ethidium bromide staining, Northern or Southern blotting, or by sequencing.

A "conservative amino acid substitution" refers to the replacement of one amino acid residue by another, chemically similar, amino acid residue. Examples of such conservative substitutions are: substitution of one hydrophobic residue (isoleucine, leucine, valine, or methionine) for another; substitution of one polar residue for another polar residue of the same charge (*e.g*., arginine for lysine; glutamic acid for aspartic acid); substitution of one aromatic amino acid (tryptophan, tyrosine, or phenylalanine) for another.

A polypeptide has "substantially the same biological activity as human calcium sensitive potassium channel β2, β3a, β3b, β3c, or β3d subunit" if that polypeptide is able to combine with a human calcium sensitive potassium channel α subunit thereby forming a functional potassium channel where the polypeptide confers upon the α subunit properties similar to those conferred by the β2, β3a, β3b, β3c, or β3d subunits and where the polypeptide has an amino acid sequence that is at least about 50% identical to SEQ.ID.NO.:2, 4, 6, 8, or 10 when measured by such standard programs as BLAST or FASTA. For example, a polypeptide that is 50% identical in amino acid sequence to β3a (SEQ.ID.NO.:4) and is able to confer upon the α subunit properties such that electrophysiological measurements of the ion channel formed by the polypeptide and the α subunit result in graphs such as those shown in Figure 7A-C for the β3a subunit and the α subunit is a polypeptide that has "substantially the same biological activity as human calcium sensitive potassium channel β3a subunit."

The present invention relates to the identification and cloning of DNAs encoding human calcium sensitive potassium channel β2, β3a, β3b, β3c, or β3d subunits, components of human calcium sensitive potassium channels. Expressed sequence tags (ESTs) (GenBank accession numbers AA904191, AI299145 and AI301175) were identified by searching databases for sequences with homology to the β1 subunit. The cDNAs encoding the ESTs were purchased and sequenced in both directions. The clone encoding AA904191 was determined to encode the entire β2 subunit, since it contained in frame stop codons 5' to the start ATG of the open reading frame and the entire open reading frame.

The β2 coding sequence was then used to search the databases for additional β subunits. Contigs were assembled from the identified ESTs and used to search the database once again. Several ESTs were identified in this iterative manner (GenBank accession numbers AA195381, AA236930, AA236968, AA279911, AA761761 and AA934876). Available cDNAs encoding these ESTs were purchased and sequenced in both directions. None of these clones were full length. Because most were isolated in a preparation of tonsils enriched for B-cells, we performed 5' RACE (rapid amplification of cDNA ends) using gene-specific oligonucleotides in the 3' untranslated region (UTR) and commercially prepared cDNA from human spleen, another tissue rich in B-cells (Clontech catalog # 7412-1), as the template. Multiple DNA fragments were amplified in this manner, cloned and sequenced in both directions. Sequencing revealed 4 subfamilies of full length clones, differing only in their 5' ends: β3a, β3b, β3c, and β3d.

The human calcium sensitive potassium channel β2, β3a, β3b, β3c, and β3d subunits of the present invention exhibit tissue specific patterns of expression. Northern blotting of mRNAs isolated from various tissues has shown that the β2 subunit is expressed predominately in uterus, heart, ovary, thyroid, fetal kidney, adrenal medulla, and pancreas; the β3a subunit is expressed predominately in heart and skeletal muscle; the β3b subunit is expressed in most tissues examined except for brain, skeletal muscle and testes. The β3c and/or β3d subunits have been found in pancreas.

The tissue specific expression patterns of the human calcium sensitive potassium channel β2, β3a, β3b, β3c, and β3d subunits support the hypothesis that these different subunits may contribute to the functional diversity of calcium sensitive potassium channels observed in different tissues. Activators and inhibitors of specific calcium sensitive potassium channels containing specific subunits may, therefore, have pharmacological efficacy in different pathological conditions, depending on the subunit composition of the calcium sensitive potassium channels involved in the specific pathological condition.

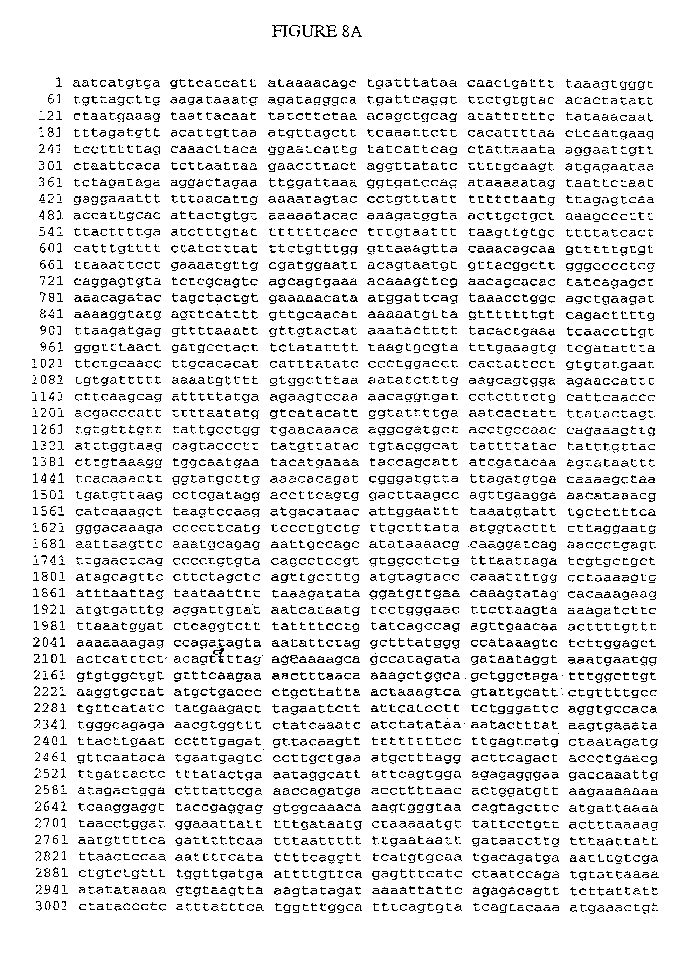

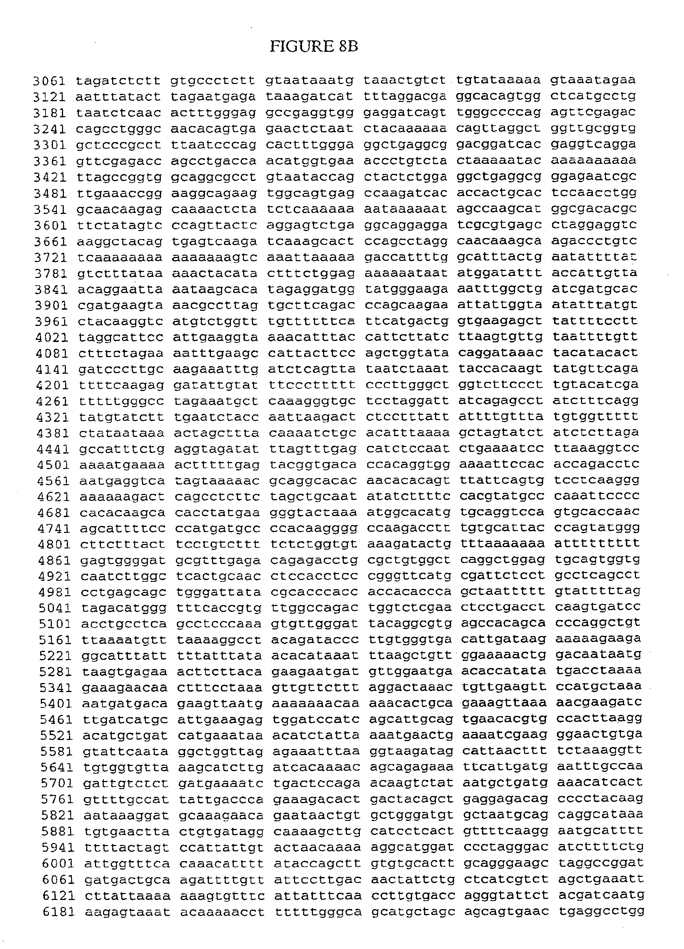

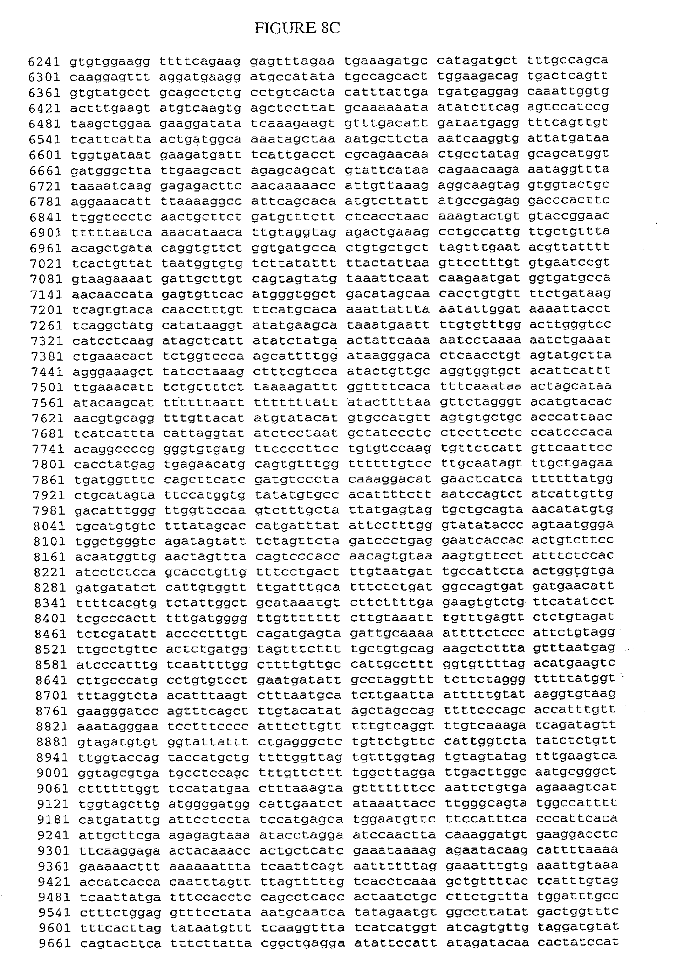

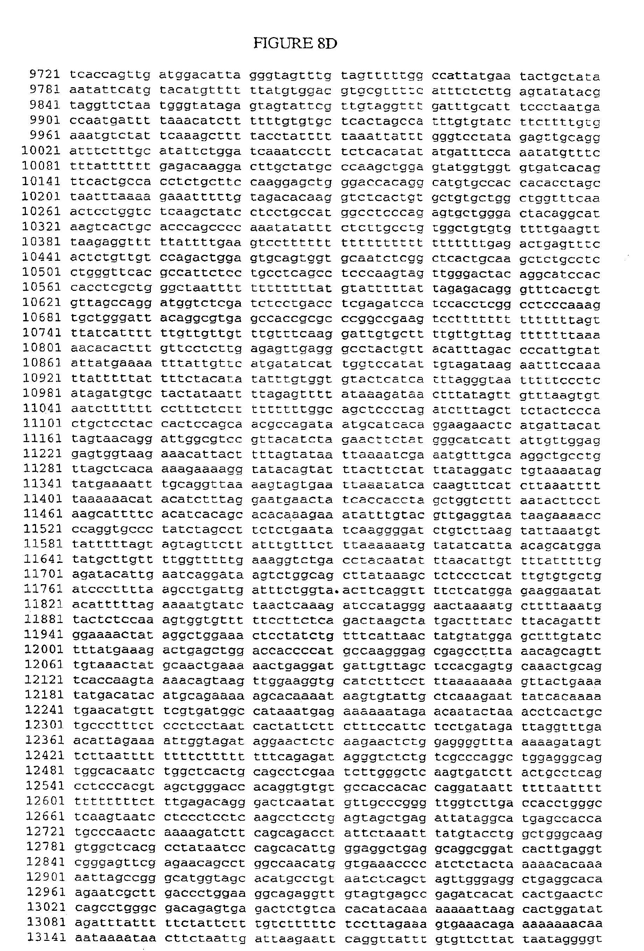

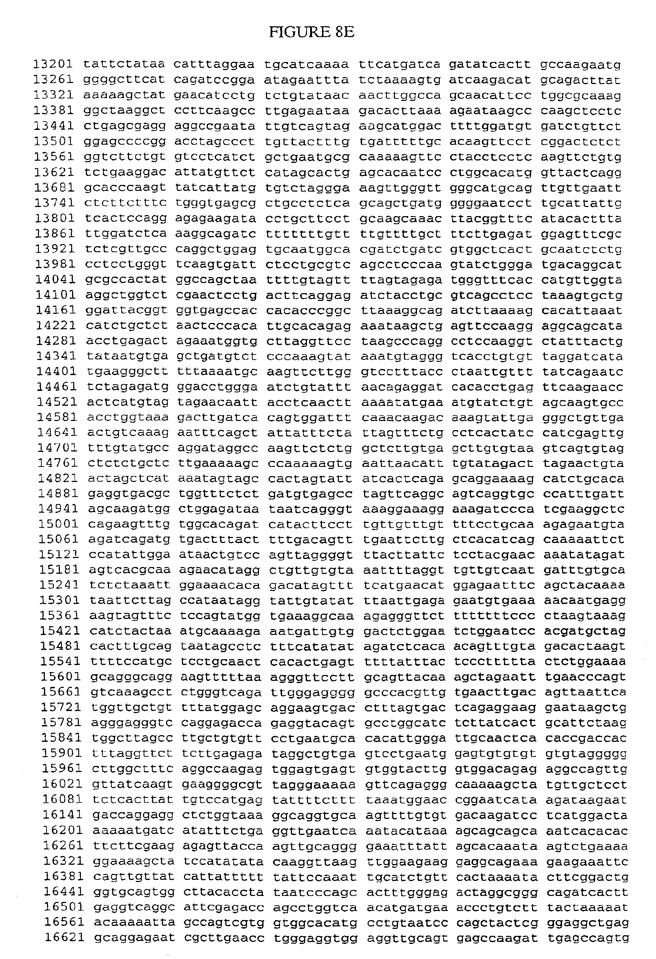

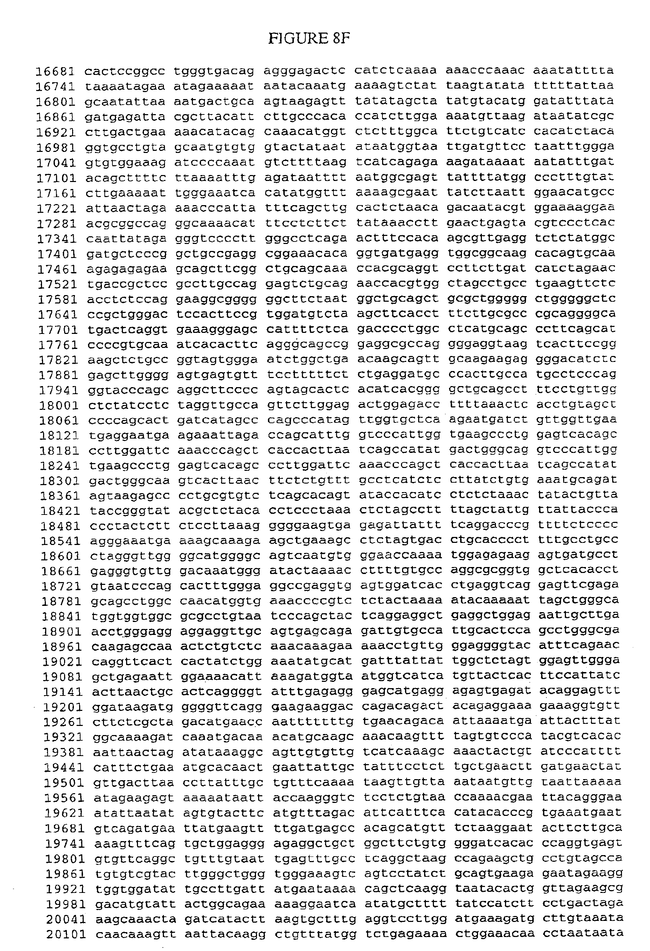

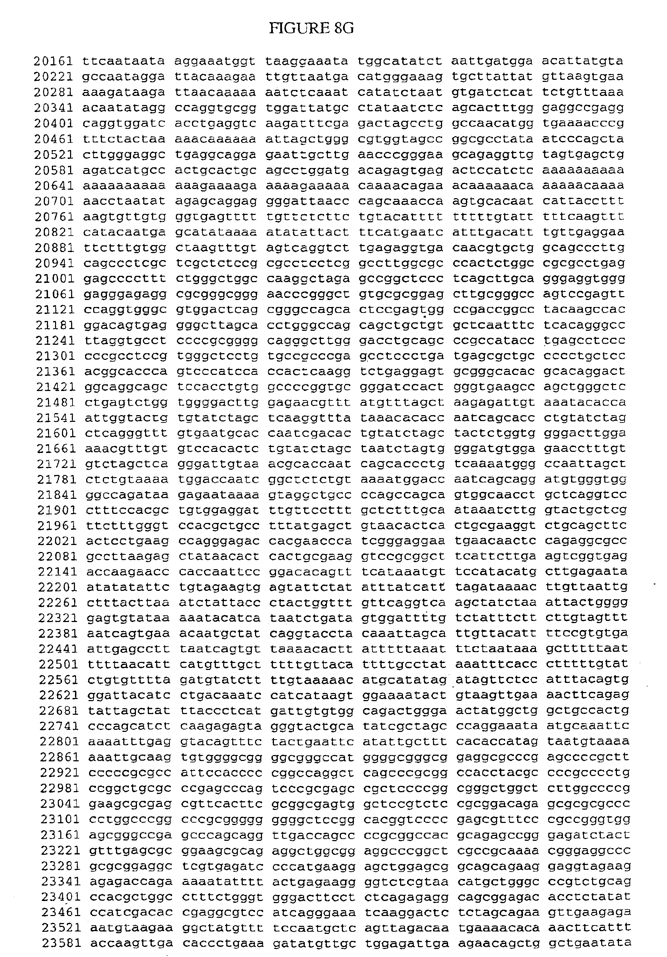

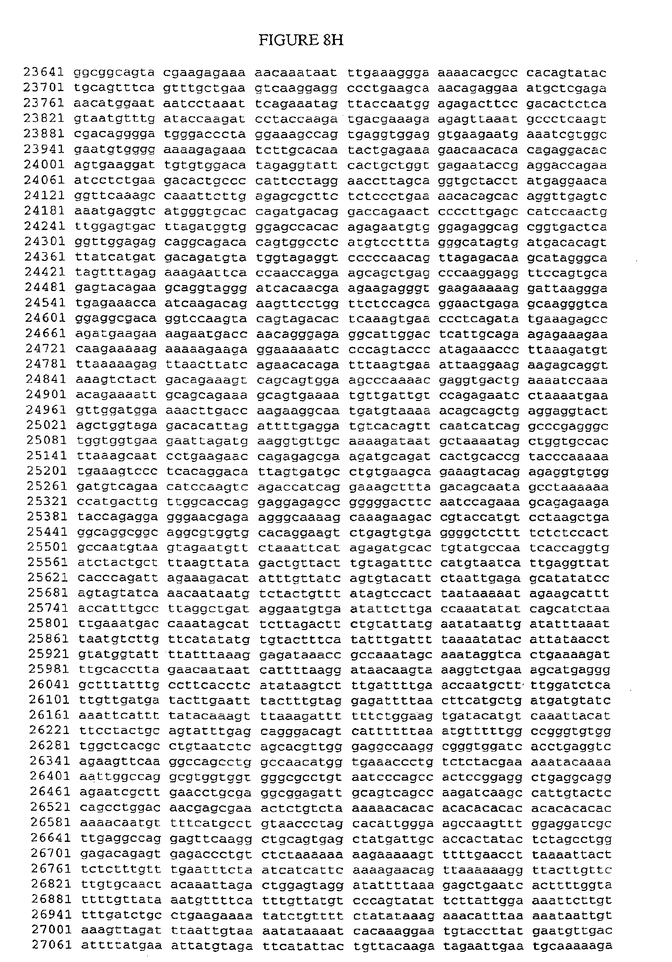

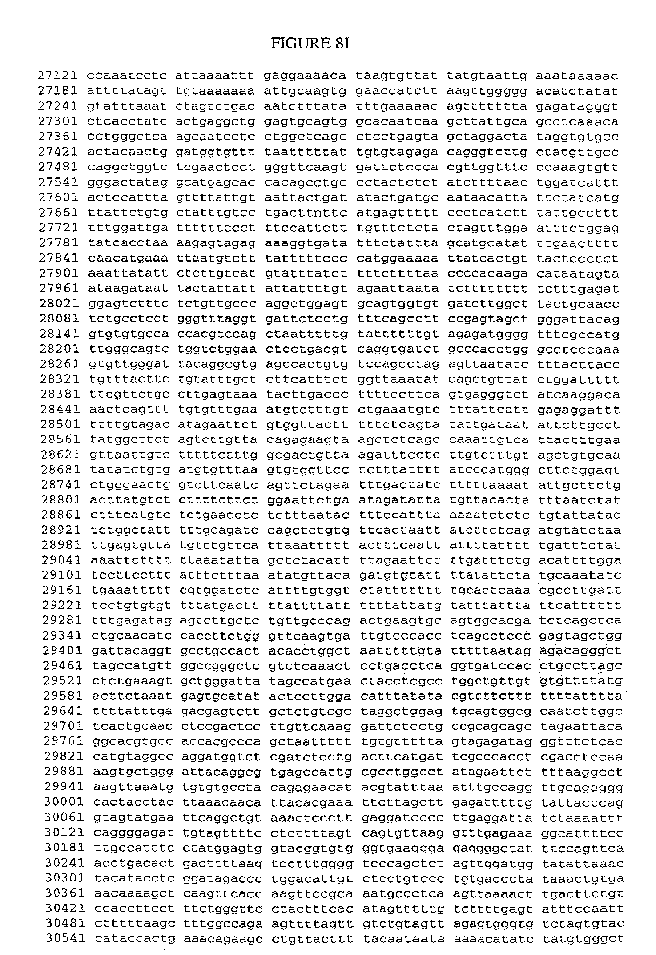

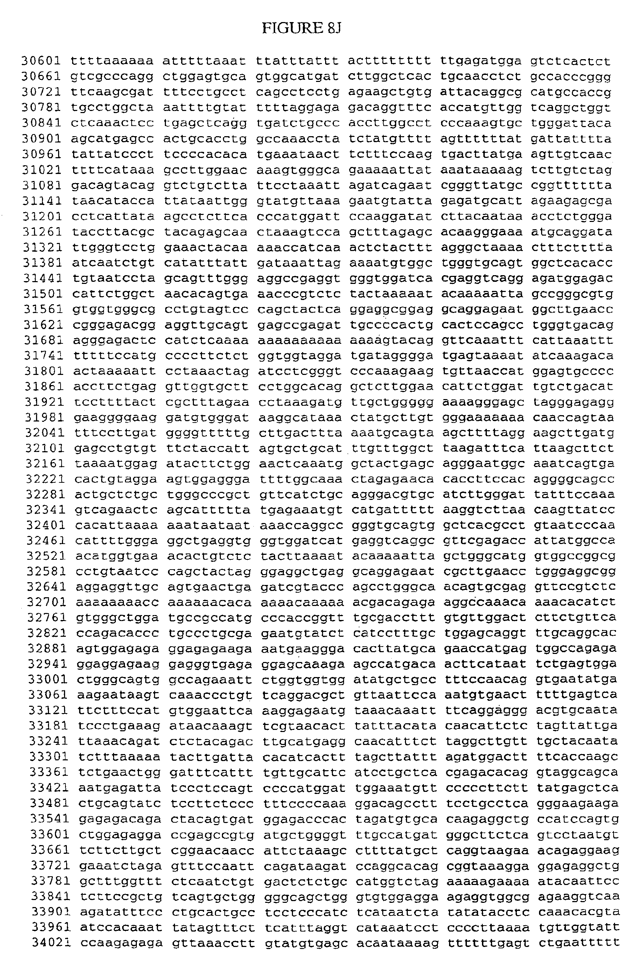

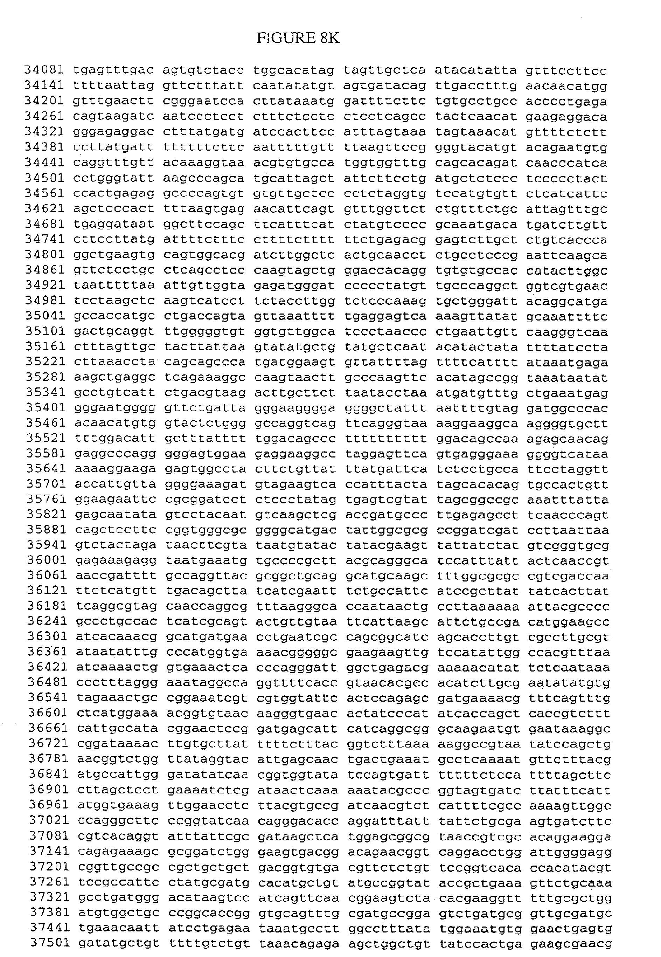

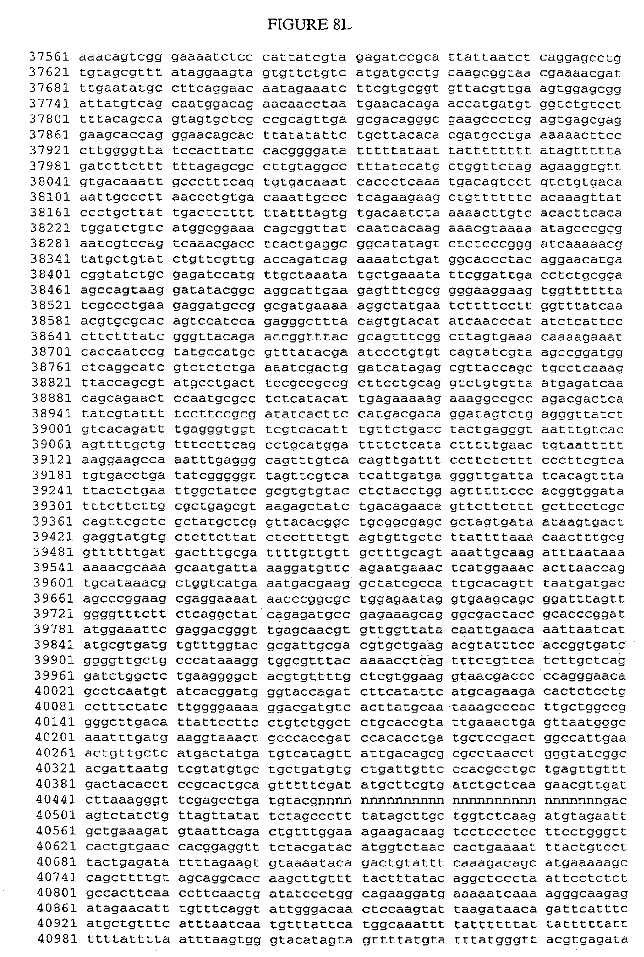

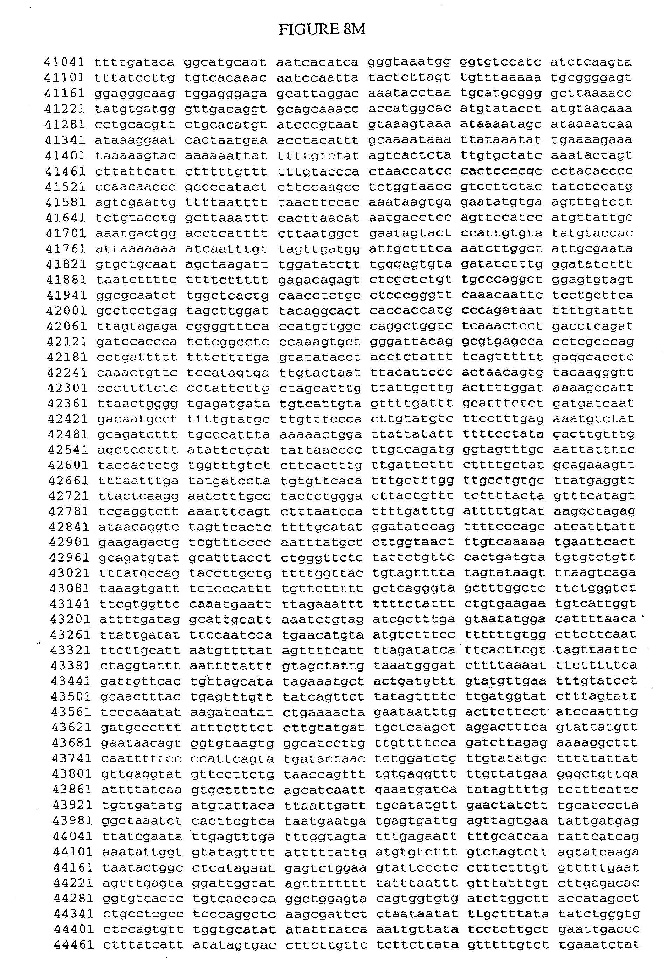

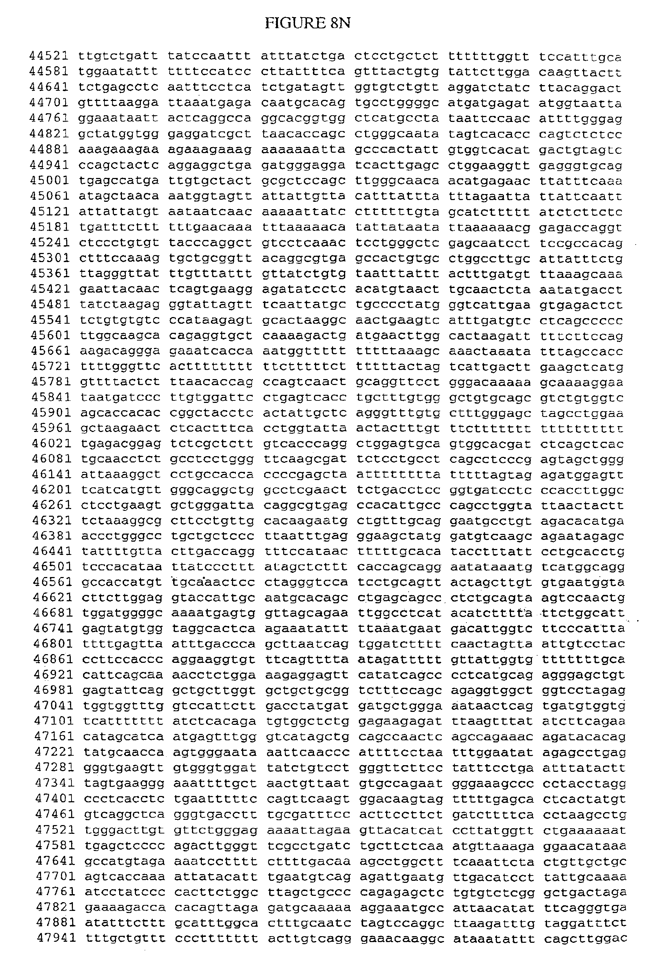

Chromosomal mapping studies have shown that both the β2 and β3 subunits map to human chromosome 3q23-ter. The β subunits of the present invention have about 30-45% amino acid sequence identity to the previously known human β1 subunit (GenBank accession no. U25138). The β2 and β3 subunits of the present invention have about 40% amino acid sequence identity to each other. The β3a, β3b, β3c, or β3d subunits differ only in their extreme N-terminal 1-20 amino acids, and are alternatively spliced variants of a single gene. Indeed, a genomic fragment of human DNA has been identified in the GenBank database that contains the 5' domains of β3a, β3b, β3c/d, and the beginning of the conserved core in a contiguous fragment (accession number AC007823.4). See Figure 8. Additionally, two bacterial artificial chromosomes (BACs) have been isolated which contain the conserved core domain. One of these BACs also contains β3c/d specific sequence. Therefore, we have identified overlapping BAC clones that together encode the entire β3 open reading frame. The β2 subunit is encoded by a separate gene.

The present invention provides DNAs encoding human calcium sensitive potassium channel β2, β3a, β3b, β3c, or β3d subunits that are substantially free from other nucleic acids. The present invention also provides isolated and/or recombinant DNA molecules encoding human calcium sensitive potassium channel β2, β3a, β3b, β3c, or β3d subunits. The present invention provides DNA molecules substantially free from other nucleic acids comprising the nucleotide sequences shown in SEQ.ID.NOs.:1, 3, 5, 7, or 9. cDNAs encoding each β3 subunit have been isolated exhibiting a sequence polymorphism, encoding either a serine or an asparagine at the amino acid position that is equivalent to position 143 of β3b. This represents amino acid 142 of the conserved core domain.

Accordingly, the present invention includes DNA substantially free from other nucleic acids as well as isolated and/or recombinant DNA encoding a polypeptide selected from the group consisting of: SEQ.1D.NO.:4; SEQ.ID.NO.:4 with an asparagine at position 163 instead of a serine; SEQ.ID.NO.:6; SEQ.ID.NO.:6 with a serine at position 143 instead of an asparagine; SEQ.ID.NO.:8; SEQ.ID.NO.:8 with an asparagine at position 161 instead of a serine; SEQ.ID.NO.:10; and SEQ.ID.NO.:10 with a serine at position 165 instead of an asparagine.

The present invention includes DNA substantially free from other nucleic acids as well as isolated and/or recombinant DNA encoding a polypeptide comprising the conserved β3 core amino acid sequence, positions 2-246 of SEQ.ID.NO.:6.

The present invention includes isolated DNA molecules as well as DNA molecules that are substantially free from other nucleic acids comprising the coding regions of SEQ.ID.NOs.:1, 3, 5, 7, and 9. Accordingly, the present invention includes isolated DNA molecules and DNA molecules substantially free from other nucleic acids having a sequence comprising positions 271 to 975 of SEQ.ID.NO.:1, positions 341 to 1171 of SEQ.ID.NO.:3, positions 796 to 1566 of SEQ.ID.NO.:5, positions 869 to 1693 of SEQ.ID.NO.:7, or positions 457 to 1293 of SEQ.ID.NO.:9.

Also included are recombinant DNA molecules having a nucleotide sequence comprising positions 271-975 of SEQ.ID.NO.:1, positions 341 to 1171 of SEQ.ID.NO.:3, positions 796 to 1566 of SEQ.ID.NO.:5, positions 869 to 1693 of SEQ.ID.NO.:7, or positions 457 to 1293 of SEQ.ID.NO.:9. The novel DNA sequences of the present invention encoding human calcium sensitive potassium channel β2, β3a, β3b, β3c, or β3d subunits, in whole or in part, can be linked with other DNA sequences, *i.e*., DNA sequences to which human calcium sensitive potassium channel β2, β3a, β3b, β3c, or β3d subunits are not naturally linked, to form "recombinant DNA molecules" encoding human calcium sensitive potassium channel β2, β3a, β3b, β3c, or β3d subunits. Such other sequences can include DNA sequences that control transcription or translation such as, *e.g*., translation initiation sequences, internal ribosome entry sites, promoters for RNA polymerase II, transcription or translation termination sequences, enhancer sequences, sequences that control replication in microorganisms, sequences that confer antibiotic resistance, or sequences that encode a polypeptide "tag" such as, *e.g*., a polyhistidine tract, the FLAG epitope, the myc epitope, GST, or maltose binding protein. The novel DNA sequences of the present invention can be inserted into vectors such as plasmids, cosmids, viral vectors, P1 artificial chromosomes, or yeast artificial chromosomes.

The present invention also includes DNA substantially free from other nucleic acids as well as isolated and/or recombinant DNA comprising genomic sequences of the human calcium sensitive potassium channel β2, β3a, β3b, β3c, or β3d subunits. The present invention includes DNA substantially free from other nucleic acids as well as isolated and/or recombinant DNA comprising SEQ.ID.NO.:20; positions 1-40,467 of SEQ.ID.NO.:20; positions 17,404-17,806 of SEQ.ID.NO.:20; positions 24,710-25,507 of SEQ.ID.NO.:20; positions 32,590-33,514 of SEQ.ID.NO.:20; positions 33,515-33,705 of SEQ.ID.NO.:20; or positions 1-17,404 of SEQ.ID.NO.:20.

Included in the present invention are DNA sequences that hybridize to at least one of SEQ.ID.NOs:1, 3, 5, 7, 9, or 20 under conditions of high stringency. By way of example, and not limitation, a procedure using conditions of high stringency is as follows: Prehybridization of filters containing DNA is carried out for 2 hr. to overnight at 65°C in buffer composed of 5X SSC, 10X Denhardt's solution, 50% Formamide, 2% SDS and 100 µg/ml denatured salmon sperm DNA. Hybridization of 32P-labelled, random primed probe is carried out in 5X SSPE, 10X Denhardts solution, 50% Formamide, 2% SDS, 100ug/ml salmon sperm DNA at 42°C overnight. Washing of filters is done in 2X SSC, 0.05% SDS at 42°C for 40 minutes, followed by 0.1X SSC, 0.05% SDS at 65°C for 40 minutes.

Other procedures using conditions of high stringency would include either a hybridization carried out in 5XSSC, 5X Denhardt's solution, 50% formamide at 42°C for 12 to 48 hours or a washing step carried out in 0.2X SSPE, 0.2% SDS at 65°C for 30 to 60 minutes.

Reagents mentioned in the foregoing procedures for carrying out high stringency hybridization are well known in the art. Details of the composition of these reagents can be found in, *e.g*., Sambrook, Fritsch, and Maniatis, 1989, Molecular Cloning: A Laboratory Manual, second edition, Cold Spring Harbor Laboratory Press. In addition to the foregoing, other conditions of high stringency which may be used are well known in the art.

The degeneracy of the genetic code is such that, for all but two amino acids, more than a single codon encodes a particular amino acid. This allows for the construction of synthetic DNA that encodes the human calcium sensitive potassium channel β2, β3a, β3b, β3c, or β3d subunit proteins where the nucleotide sequence of the synthetic DNA differs significantly from the nucleotide sequences of SEQ.ID.NOs:1, 3, 5, 7, or 9 but still encodes the same human calcium sensitive potassium channel β2, β3a, β3b, β3c, or β3d subunit proteins as SEQ.ID.NOs:2, 4, 6, 8, or 10. Such synthetic DNAs are intended to be within the scope of the present invention.

Mutated forms of SEQ.ID.NOs:1, 3, 5, 7, or 9 are intended to be within the scope of the present invention. In particular, mutated forms of SEQ.ID.NOs:1, 3, 5, 7, or 9 which encode proteins that either do not interact with an α subunit or which when combined with α subunits give rise to calcium sensitive potassium channels having altered voltage dependence, calcium sensitivity, current kinetics (such as activation, inactivation or deactivation), or pharmacologic properties as compared to wild-type calcium sensitive potassium channels are within the scope of the present invention. Such mutant forms can differ from SEQ.ID.NOs:1, 3, 5, 7, or 9 by having nucleotide deletions, substitutions, or additions.

Also intended to be within the scope of the present invention are RNA molecules having sequences corresponding to SEQ.ID.NOs:1, 3,5, 7, or 9. Antisense nucleotides, DNA or RNA, that are the reverse complements of SEQ.ID.NOs:1, 3, 5, 7, or 9, or portions thereof, are also within the scope of the present invention. In addition, polynucleotides based on SEQ.ID.NOs:1, 3, 5, 7, or 9 in which a small number of positions are substituted with non-natural or modified nucleotides such as inosine, methyl-cytosine, or deaza-guanosine are intended to be within the scope of the present invention. Polynucleotides of the present invention can also include sequences based on SEQ.ID.NOs:1, 3, 5, 7, or 9 but in which non-natural linkages between the nucleotides are present. Such non-natural linkages can be, *e.g*., methylphosphonates, phosphorothioates, phosphorodithionates, phosphoroamidites, and phosphate esters. Polynucleotides of the present invention can also include sequences based on SEQ.ID.NOs:1, 3, 5, 7, or 9 but having de-phospho linkages as bridges between nucleotides, *e.g*., siloxane, carbonate, carboxymethyl ester, acetamidate, carbamate, and thioether bridges. Other internucleotide linkages that can be present include N-vinyl, methacryloxyethyl, methacrylamide, or ethyleneimine linkages. Peptide nucleic acids based upon SEQ.IDD.NOs:1, 3, 5, 7, or 9 are also included in the present invention.

Another aspect of the present invention includes host cells that have been engineered to contain and/or express DNA sequences encoding human calcium sensitive potassium channel β2, β3a, β3b, β3c, or β3d subunit proteins. Such recombinant host cells can be cultured under suitable conditions to produce human calcium sensitive potassium channel β2, β3a, β3b, β3c, or β3d subunit proteins. Such recombinant host cells are also useful in the methods of identifying activators and inhibitors of calcium sensitive potassium channels described herein. An expression vector containing DNA encoding human calcium sensitive potassium channel β2, β3a, β3b, β3c, or β3d subunit proteins can be used for the expression of human calcium sensitive potassium channel β2, β3a, β3b, β3c, or β3d subunit proteins in a recombinant host cell. Recombinant host cells may be prokaryotic or eukaryotic, including but not limited to, bacteria such as *E*. *coli,* fungal cells such as yeast, mammalian cells including, but not limited to, cell lines of human, bovine, porcine, monkey and rodent origin, amphibian cells such as *Xenopus* oocytes, and insect cells including but not limited to *Drosophila* and silkworm derived cell lines. Cells and cell lines which are suitable for recombinant expression of human calcium sensitive potassium channel β2, β3a, β3b, β3c, or β3d subunit proteins and which are widely available, include but are not limited to, L cells L-M(TK⁻) (ATCC CCL 1.3), L cells L-M (ATCC CCL 1.2), 293 (ATCC CRL 1573), Raji (ATCC CCL 86), CV-1 (ATCC CCL 70), COS-1 (ATCC CRL 1650), COS-7 (ATCC CRL 1651), CHO-K1 (ATCC CCL 61), 3T3 (ATCC CCL 92), NIH/3T3 (ATCC CRL 1658), HeLa (ATCC CCL 2), C127I (ATCC CRL 1616), BS-C-1 (ATCC CCL 26), MRC-5 (ATCC CCL 171), CPAE (ATCC CCL 209), Saos-2 (ATCC HTB-85), ARPE-19 human retinal pigment epithelium (ATCC CRL-2302), *Xenopus* melanophores, and *Xenopus* oocytes.

A variety of mammalian expression vectors can be used to express recombinant human calcium sensitive potassium channel β2, β3a, β3b, β3c, or β3d subunit proteins in mammalian cells. Commercially available mammalian expression vectors which are suitable include, but are not limited to, pMClneo (Stratagene), pSG5 (Stratagene), pcDNAI and pcDNAIamp, pcDNA3, pcDNA3.1, pCR3.1 (Invitrogen), EBO-pSV2-neo (ATCC 37593), pBPV-1(8-2) (ATCC 37110), pdBPV-MMTneo(342-12) (ATCC 37224), pRSVgpt (ATCC 37199), pRSVneo (ATCC 37198), pIZD35 (ATCC 37565), and pSV2-dhfr (ATCC 37146). Another suitable vector is the PT7TS oocyte expression vector.

Following expression in recombinant cells, human calcium sensitive potassium channel β2, β3a, β3b, β3c, or β3d subunit proteins can be purified by conventional techniques to a level that is substantially free from other proteins. Techniques that can be used include ammonium sulfate precipitation, hydrophobic or hydrophilic interaction chromatography, ion exchange chromatography, affinity chromatography, phosphocellulose chromatography, size exclusion chromatography, preparative gel electrophoresis, and alcohol precipitation, In some cases, it may be advantageous to employ protein denaturing and/or refolding steps in addition to such techniques.

Certain voltage-gated potassium channel subunits have been found to require the expression of other voltage-gated potassium channel subunits in order to be properly expressed at high levels and inserted in membranes. For example, co-expression of KCNQ3 appears to enhance the expression of KCNQ2 in *Xenopus* oocytes (Wang et al., 1998, Science 282:1890-1893). Also, some voltage-gated potassium channel α subunits require other related αsubunits (Jegla and Salkoff, 1997, J. Neurosci. 17:32-44) or Kνβ2 subunits (Shi et al., 1995, Neuron 16:843-852). Accordingly, the recombinant expression of the human calcium sensitive potassium channel β2, β3a, β3b, β3c, or β3d subunit proteins may, under certain circumstances, benefit from the co-expression of other proteins and such co-expression is intended to be within the scope of the present invention. A particularly preferred form of co-expression is the co-expression of a human calcium sensitive potassium channel β2, β3a, β3b, β3c, or β3d subunit protein (or combinations thereof) with a human calcium sensitive potassium channel α subunit protein. Such co-expression can be effected by transfecting an expression vector encoding a human calcium sensitive potassium channel β2, β3a, β3b, β3c, or β3d subunit protein into a cell that naturally expresses a human calcium sensitive potassium channel α subunit protein. Alternatively, an expression vector encoding a human calcium sensitive potassium channel β2, β3a, β3b, β3c, or β3d subunit protein can be transfected into a cell in which an expression vector encoding a human calcium sensitive potassium channel α subunit protein has also been transfected. Preferably, such a cell does not naturally express human calcium sensitive potassium channel α or β subunits.

The present invention includes human calcium sensitive potassium channel β2, β3a, β3b, β3c, and β3d subunit proteins substantially free from other proteins. The deduced amino acid sequences of the full-length human calcium sensitive potassium channel β2, β3a, β3b, β3c, and β3d subunit proteins are shown in SEQ.ID.NOs.:2, 4, 6, 8, and 10, respectively. Thus, the present invention includes human calcium sensitive potassium channel β2, β3a, β3b, β3c, and β3d subunit proteins substantially free from other proteins having the amino acid sequences SEQ.ID.NO.:2, SEQ.ID.NO.:4; SEQ.ID.NO.:4 with an asparagine at position 163 instead of a serine; SEQ.ID.NO.:6; SEQ.ID.NO.:6 with a serine at position 143 instead of an asparagine; SEQ.ID.NO.:8; SEQ.ID.NO.:8 with an asparagine at position 161 instead of a serine; SEQ.ID.NO.:10; and SEQ.ID.NO.;10 with a serine at position 165 instead of an asparagine. The present invention also includes isolated human calcium sensitive potassium channel β2, β3a, β3b, β3c, and β3d subunit proteins having the amino acid sequences SEQ.ID.NO.:2, SEQ.ID.NO.:4; SEQ.ID.NO.:4 with an asparagine at position 163 instead of a serine; SEQ.ID.NO.:6; SEQ.ID.NO.:6 with a serine at position 143 instead of an asparagine; SEQ.ID.NO.:8; SEQ.ID.NO.:8 with an asparagine at position 161 instead of a serine; SEQ.ID.NO.:10; and SEQ.ID.NO.:10 with a serine at position 165 instead of an asparagine.

Mutated forms of human calcium sensitive potassium channel β2, β3a, β3b, β3c, and β3d subunit proteins are intended to be within the scope of the present invention. In particular, mutated forms of SEQ.ID.NOs:2, 4, 6, 8, and 10 that give rise to calcium sensitive potassium channels having altered electrophysiological or pharmacological properties when combined with α subunits are within the scope of the present invention.

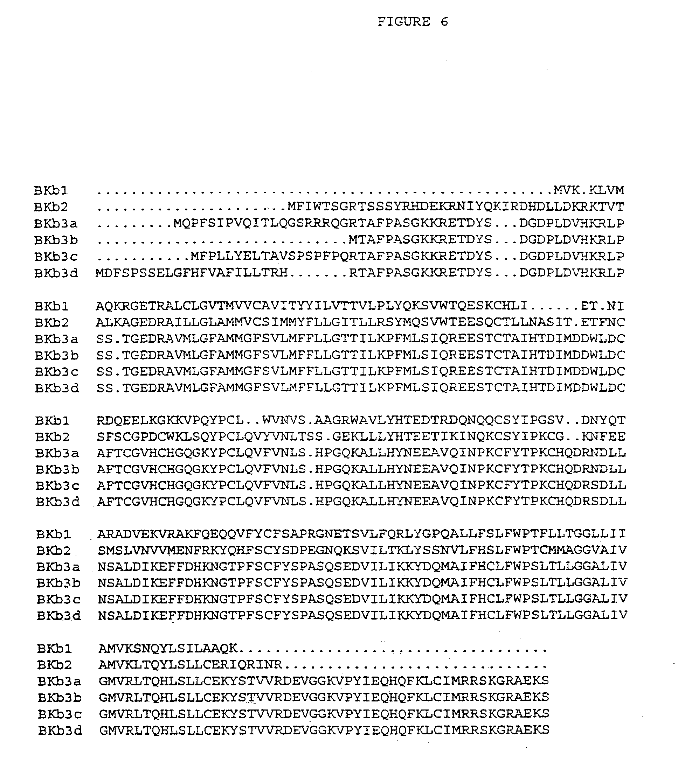

As with many proteins, it is possible to modify many of the amino acids of human calcium sensitive potassium channel β2, β3a, β3b, β3c, or β3d subunit proteins and still retain substantially the same biological activity as for the original proteins. Thus, the present invention includes modified human calcium sensitive potassium channel β2, β3a, β3b, β3c, and β3d subunit proteins which have amino acid deletions, additions, or substitutions but that still retain substantially the same biological activity as naturally occurring human calcium sensitive potassium channel β2, β3a, β3b, β3c, or β3d subunit proteins. It is generally accepted that single amino acid substitutions do not usually alter the biological activity of a protein (see, *e.*g., Molecular Biology of the Gene, Watson *et al.,* 1987, Fourth Ed., The Benjamin/Cummings Publishing Co., Inc., page 226; and Cunningham & Wells, 1989, Science 244:1081-1085). Accordingly, the present invention includes polypeptides where one amino acid substitution has been made in SEQ.ID.NOs:2, 4, 6, 8, or 10 wherein the polypeptides still retain substantially the same biological activity as naturally occurring human calcium sensitive potassium channel β2, β3a, β3b, β3c, or β3d subunit proteins. The present invention also includes polypeptides where two or more amino acid substitutions have been made in SEQ.ID.NOs:2, 4, 6, 8, or 10 wherein the polypeptides still retain substantially the same biological activity as naturally occurring human calcium sensitive potassium channel β2, β3a, β3b, β3c, or β3d subunit proteins. In particular, the present invention includes embodiments where the above-described substitutions are conservative substitutions. In particular, the present invention includes embodiments where the above-described substitutions do not occur in conserved positions. Conserved positions are those positions in which the human calcium sensitive potassium channel β1, β2, and any of the β3 subunits all have the same amino acid (see Figure 6).

The human calcium sensitive potassium channel β2, β3a, β3b, β3c, or β3d subunit proteins of the present invention may contain post-translational modifications, *e.g*., covalently linked carbohydrate, phosphorylation, myristoylation, palmyltoylation, *etc*.

The present invention also includes chimeric human calcium sensitive potassium channel β2, β3a, β3b, β3c, or β3d subunit proteins. Chimeric human calcium sensitive potassium channel β2, β3a, β3b, β3c, or β3d subunit proteins consist of a contiguous polypeptide sequence of at least a portion of a human calcium sensitive potassium channel β2, β3a, β3b, β3c, or β3d subunit protein fused to a polypeptide sequence that is not from a human calcium sensitive potassium channel β2, β3a, β3b, β3c, or β3d subunit protein.

The present invention also includes isolated human calcium sensitive potassium channel β2, β3a, β3b, β3c, or β3d subunit proteins and DNA encoding these isolated subunits. Use of the term "isolated" indicates that the human calcium sensitive potassium channel β2, β3a, β3b, β3c, or β3d subunit protein or DNA has been removed from its normal cellular environment. Thus, an isolated human calcium sensitive potassium channel β2, β3a, β3b, β3c, or β3d subunit protein may be in a cell-free solution or placed in a different cellular environment from that in which it occurs naturally. The term isolated does not imply that an isolated human calcium sensitive potassium channel β2, β3a, β3b, β3c, or β3d subunit protein is the only protein present, but instead means that the isolated human calcium sensitive potassium channel β2, β3a, β3b, β3c, or β3d subunit protein is at least 95% free of non-amino acid material (*e.g*., nucleic acids, lipids, carbohydrates) naturally associated with the human calcium sensitive potassium channel β2, β3a, β3b, β3c, or β3d subunit protein. Thus, a human calcium sensitive potassium channel β2, β3a, β3b, β3c, or β3d subunit protein that is expressed in bacteria or even in eukaryotic cells which do not naturally *(i.e.,* without human intervention) express it through recombinant means is an "isolated human calcium sensitive potassium channel β2, β3a, β3b, β3c, or β3d subunit protein."

It is known that certain potassium channels subunits can interact to form heteromeric structures resulting in functional potassium channels. For example, KCNQ2 and KCNQ3 can assemble to form a heteromeric functional potassium channel (Wang et al., 1998, Science 282:1890-1893). Accordingly, it is believed likely that the human calcium sensitive potassium channel β2, β3a, β3b, β3c, or β3d subunit proteins of the present invention will also be able to form heteromeric structures with other proteins where such heteromeric structures constitute functional potassium channels. Thus, the present invention includes such heteromers comprising human calcium sensitive potassium channel β2, β3a, β3b, β3c, or β3d subunit proteins. Preferred heteromers are those in which the human calcium sensitive potassium channel β2, β3a, β3b, β3c, or β3d subunit proteins of the present invention forms heteromers with calcium sensitive potassium channel α subunits.

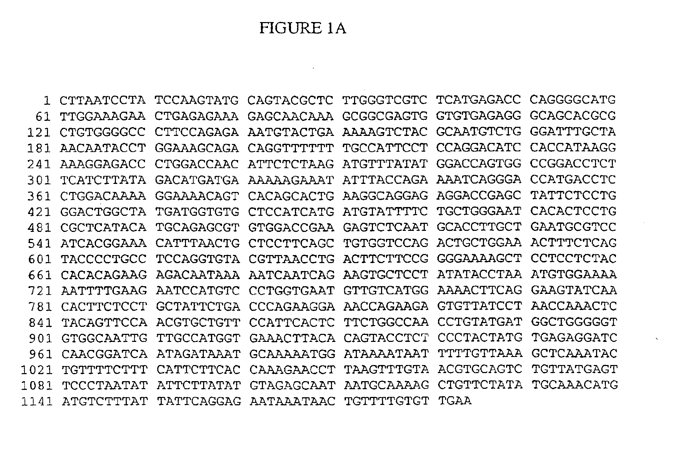

DNA encoding the human calcium sensitive potassium channel β2, β3a, β3b, β3c, or β3d subunit proteins can be obtained by methods well known in the art. For example, a cDNA fragment encoding full-length human calcium sensitive potassium channel β2 subunit protein can be isolated from human uterus, ovary or pancreas cDNA by using the polymerase chain reaction (PCR) employing suitable primer pairs. Such primer pairs can be selected based upon the DNA sequence encoding the human calcium sensitive potassium channel β2 subunit protein shown in Figure 1A as SEQ.ID.NO.:1. Suitable primer pairs would be, *e.g.:* 5'-AAG ATG TTT ATA TGG ACC AGT GGC-3' (SEQ.ID.NO.:12) and

5'-ACT CAT AAC AGA CTG CAC GTT AC-3' (SEQ.ID.NO.:13).

The above and subsequent primers are meant to be illustrative only; one skilled in the art would readily be able to design other suitable primers based upon SEQ.ID.NO.:1. Such primers could be produced by methods of oligonucleotide synthesis that are well known in the art.

In a similar manner, PCR primers can be selected and produced for the other human calcium sensitive potassium channel subunit proteins of the present invention. For example, for the human calcium sensitive potassium channel β3a subunit, suitable primer pairs would be, *e.g*.: 5'-GTC ATG CAG CCC TTC AGC ATC CC-3' (SEQ.ID.NO.:14) and

5'-TTG CAG AAA TCA CAG ACA TCT GAA-3' (SEQ.ID.NO.:15).

A suitable cDNA template from which the human calcium sensitive potassium channel β3a subunit can be isolated is human heart, skeletal muscle or spleen cDNA.

For the human calcium sensitive potassium channel β3b subunit, suitable primer pairs would be, *e.g*.: 5'-GCA ATG ACA GCC TTT CCT GCC TC-3' (SEQ.ID.NO.:16) and

5’-TTG CAG AAA TCA CAG ACA TCT GAA-3’ (SEQ.ID.NO.:15).

A suitable cDNA template from which the human calcium sensitive potassium channel β3b subunit can be isolated is human spleen cDNA.

For the human calcium sensitive potassium channel β3c subunit, suitable primer pairs would be, *e.g*.: 5'-GAA ATG TTC CCC CTT CTT TAT GAG-3' (SEQ.ID.NO.:17) and

5'-TTG CAG AAA TCA CAG ACA TCT GAA-3' (SEQ.ID.NO.:15).

A suitable cDNA template from which the human calcium sensitive potassium channel β3c subunit can be isolated is human pancreas or spleen cDNA.

For the human calcium sensitive potassium channel β3d subunit, suitable primer pairs would be, *e.g*.: 5'-GAG ATG GAC TTT TCA CCA AGC TCT-3' (SEQ.ID.NO.:18) and

5'-TTG CAG AAA TCA CAG ACA TCT GAA-3' (SEQ.ID.NO.:15).

A suitable cDNA template from which the human calcium sensitive potassium channel β3d subunit can be isolated is human pancreas or spleen cDNA.

PCR reactions can be carried out with a variety of thermostable enzymes including but not limited to AmpliTaq, AmpliTaq Gold, or Vent polymerase. For AmpliTaq, reactions can be carried out in 10 mM Tris-Cl, pH 8.3, 2.0 mM MgCl₂, 200 µM for each dNTP, 50 mM KCl, 0.2 µM for each primer, 10 ng of DNA template, 0.05 units/µl of AmpliTaq. The reactions are heated at 95°C for 3 minutes and then cycled 25 times using the cycling parameters of 95°C, 20 seconds, 62°C, 20 seconds, 72°C, 3 minutes. In addition to these conditions, a variety of suitable PCR protocols can be found in PCR Primer, A Laboratory Manual, edited by C.W. Dieffenbach and G.S. Dveksler, 1995, Cold Spring Harbor Laboratory Press; or PCR Protocols: A Guide to Methods and Applications, Michael *et al.,* eds., 1990, Academic Press.

Since the calcium sensitive potassium channel subunits of the present invention are highly homologous to one another, and to other potassium channel subunits, it is desirable to sequence the clones obtained by the herein-described methods, in order to verify that the desired calcium sensitive potassium channel β subunits have in fact been obtained.

By these methods, cDNA clones encoding the human calcium sensitive potassium channel β2, β3a, β3b, β3c, or β3d subunit proteins can be obtained. These cDNA clones can be cloned into suitable cloning vectors or expression vectors, *e.g*., the mammalian expression vector pcDNA3.1 (Invitrogen, San Diego, CA). Human calcium sensitive potassium channel β2, β3a, β3b, β3c, or β3d subunit proteins can then be produced by transfecting expression vectors encoding the subunits or portions thereof into suitable host cells and growing the host cells under appropriate conditions. Human calcium sensitive potassium channel β2, β3a, β3b, β3c, or β3d subunit proteins can then be isolated by methods well known in the art.

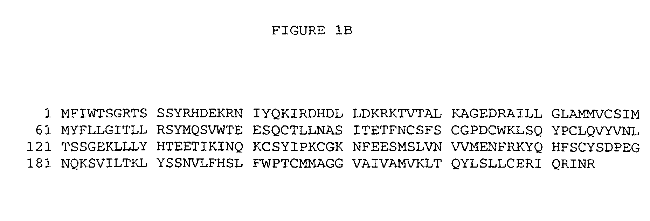

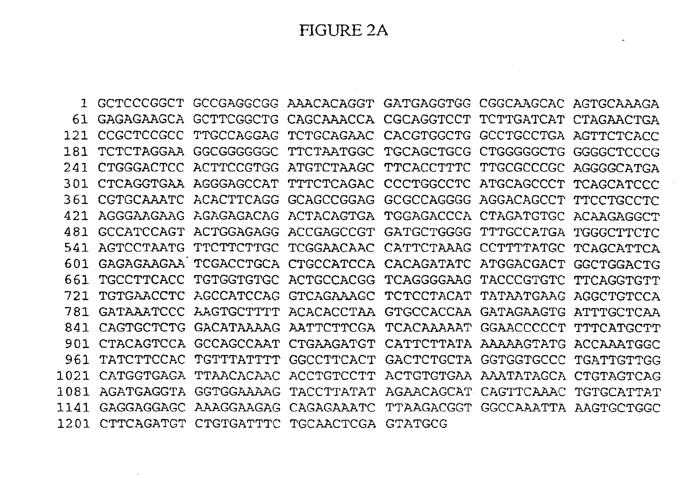

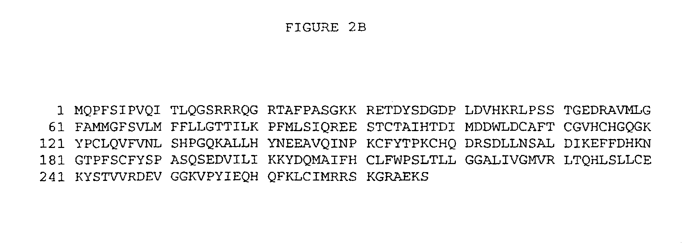

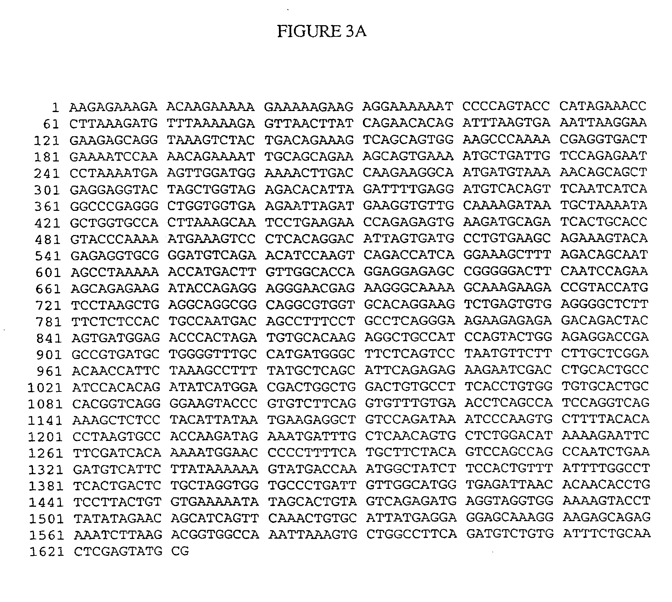

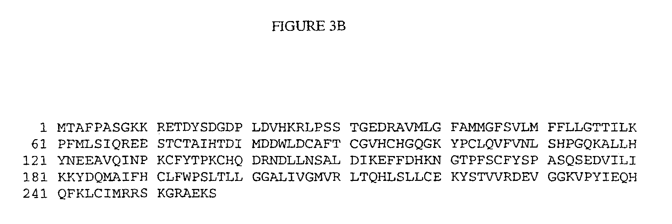

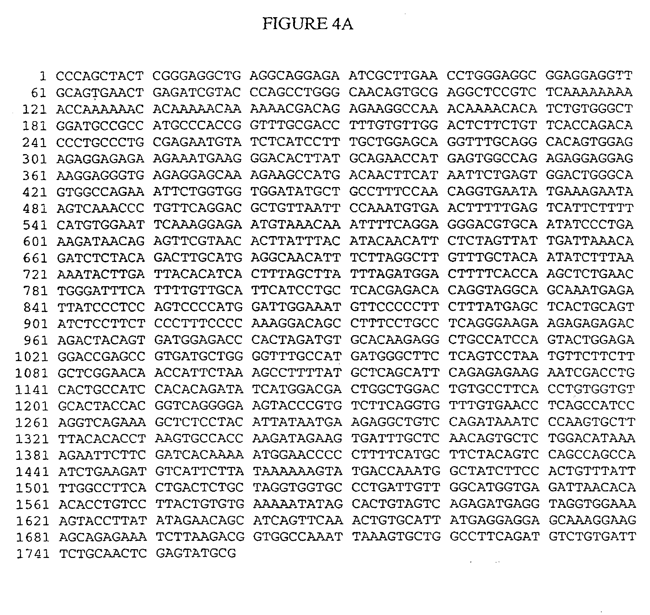

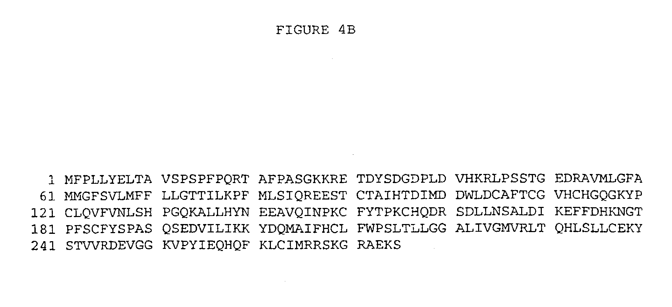

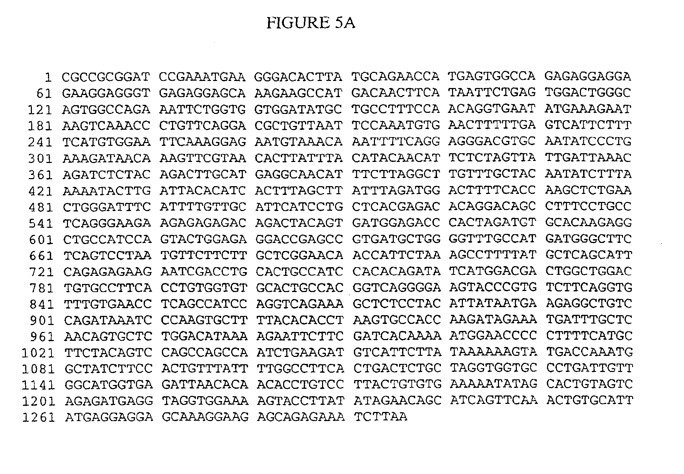

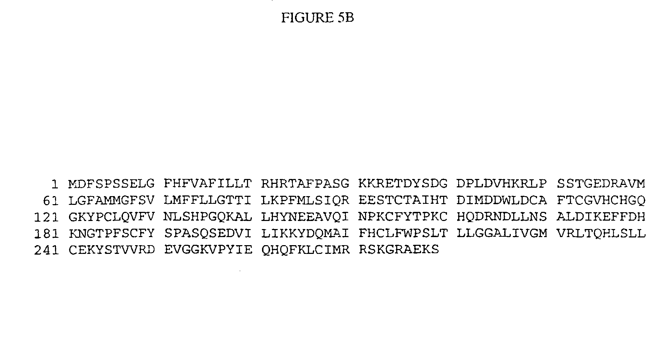

As an alternative to the above-described PCR methods, cDNA clones encoding the human calcium sensitive potassium channel β2, β3a, β3b, β3c, or β3d subunit proteins can be isolated from cDNA libraries using as a probe oligonucleotides specific for each human calcium sensitive potassium channel β2, β3a, β3b, β3c, or β3d subunit and methods well known in the art for screening cDNA libraries with oligonucleotide probes. Such methods are described in, *e.g.,* Sambrook *et al.,* 1989, *Molecular Cloning: A Laboratory Manual;* Cold Spring Harbor Laboratory, Cold Spring Harbor, New York; Glover, D.M. (ed.), 1985, *DNA Cloning: A Practical Approach,* MRL Press, Ltd., Oxford, U.K., Vol. I, II. Oligonucleotides that are specific for particular human calcium sensitive potassium channel β2, β3a, β3b, β3c, or β3d subunits and that can be used to screen cDNA libraries can be readily designed based upon the DNA sequences shown in Figures 1-5 and can be synthesized by methods well-known in the art.

Genomic clones containing the human calcium sensitive potassium channel β2, β3a, β3b, β3c, or β3d subunit genes can be obtained from commercially available human PAC, YAC, or BAC libraries available from Research Genetics, Huntsville, AL. Alternatively, one may prepare genomic libraries, *e.g.,* in P1 artificial chromosome vectors, from which genomic clones containing the human calcium sensitive potassium channel β2, β3a, β3b, β3c, or β3d subunit genes can be isolated, using probes based upon the human calcium sensitive potassium channel β2, β3a, β3b, β3c, or β3d subunit DNA sequences disclosed herein. Methods of preparing such libraries are known in the art (see, *e.g*., Ioannou *et al*., 1994, Nature Genet. 6:84-89).

The novel DNA sequences of the present invention can be used in various diagnostic methods. The present invention provides diagnostic methods for determining whether a patient carries a mutation in one or more of the human calcium sensitive potassium channel β2, β3a, β3b, β3c, or β3d subunit genes. In broad terms, such methods comprise determining the DNA sequence of a region in or near one or more of the human calcium sensitive potassium channel β2, β3a, β3b, β3c, or β3d subunit genes from the patient and comparing that sequence to the sequence from the corresponding region of the human calcium sensitive potassium channel β2, β3a, β3b, β3c, or β3d subunit genes from a non-affected person, *i.e*., a person who does not have the condition which is being diagnosed, where a difference in sequence between the DNA sequence of the gene from the patient and the DNA sequence of the gene from the non-affected person indicates that the patient has a mutation in one or more of the human calcium sensitive potassium channel β2, β3a, β3b, β3c, or β3d subunit genes.

The present invention also provides oligonucleotide probes, based upon the sequences of SEQ.ID.NOs:1, 3, 5, 7, 9, or 20 that can be used in diagnostic methods to identify patients having mutated forms of human calcium sensitive potassium channel β2, β3a, β3b, β3c, or β3d subunits, to determine the level of expression of RNA encoding human calcium sensitive potassium channel β2, β3a, β3b, β3c, or β3d subunits, or to isolate genes homologous to human calcium sensitive potassium channel β2, β3a, β3b, β3c, or β3d subunits. In particular, the present invention includes DNA oligonucleotides comprising at least about 10, 15, or 18 contiguous nucleotides of a sequence selected from the group consisting of: SEQ.ID.NOs:1, 3, 5, 7, 9, and 20 where the oligonucleotide probe comprises no stretch of contiguous nucleotides longer than 5 of a sequence selected from the group consisting of: SEQ.ID.NOs:1, 3, 5, 7, 9, and 20 other than the said at least about 10, 15, or 18 contiguous nucleotides. The oligonucleotides can be substantially free from other nucleic acids. Also provided by the present invention are corresponding RNA oligonucleotides. The DNA or RNA oligonucleotide can be packaged in kits for use as probes.

The present invention makes possible the recombinant expression of the human calcium sensitive potassium channel β2, β3a, β3b, β3c, or β3d subunit proteins in various cell types.

The β2, β3a, β3b, β3c, and β3d subunits of the human calcium sensitive potassium channel have been expressed in *Xenopus* oocytes, both by themselves and in combination with an α subunit of a large-conductance calcium-sensitive potassium channel (maxi-K channel). The β subunits do not express currents on their own. However, when co-expressed with the α subunit, the β2, β3a, and β3c subunits induce inactivation of calcium sensitive potassium currents (Figure 7). The rates of inactivation produced by β2, β3a and β3c are dependent upon voltage and internal calcium concentration; inactivation time constants reach a maximum at high depolarizations and high micromolar calcium for β2, β3a and β3c, τ_{inact} ~ 30-40 ms at 80 mV with 30 µM intracellular Ca²⁺. Measurements of current-voltage dependence obtained in the presence of micromolar intracellular Ca²⁺ demonstrate that β2 subunits induce a large shift in the voltage dependence of activation (~80 mV towards negative potentials, with 30µM Ca²⁺ in the bath; Figure 7B). This modulatory effect is similar to the one previously described for β1 subunits, which do not induce inactivation. (McManus et al., 1995, Neuron 14:645-650). In contrast, β3a, β3b, β3c, and β3d subunits do not shift the voltage dependence when compared to channels containing only α subunits (Figure 7B).

The present invention also makes possible the development of assays that measure the biological activity of calcium sensitive potassium channels containing human calcium sensitive potassium channel β2, β3a, β3b, β3c, or β3d subunit proteins. Such assays using recombinantly expressed human calcium sensitive potassium channel β2, β3a, β3b, β3c, or β3d subunit proteins are especially of interest. Such assays can be used to screen libraries of compounds or other sources of compounds to identify compounds that are activators or inhibitors of the activity of calcium sensitive potassium channels containing human calcium sensitive potassium channel β2, β3a, β3b, β3c, or β3d subunit proteins. Such identified compounds can serve as "leads" for the development of pharmaceuticals that can be used to treat patients having diseases in which it is beneficial to enhance or suppress calcium sensitive potassium channel activity.

In versions of the above-described assays, calcium sensitive potassium channels containing mutant human calcium sensitive potassium channel β2, β3a, β3b, β3c, or β3d subunit proteins are used and inhibitors or activators of the activity of the mutant calcium sensitive potassium channels are identified.

Preferred cell lines for recombinant expression of human calcium sensitive potassium channel β2, β3a, β3b, β3c, or β3d subunit proteins are those which do not express endogenous potassium channels (*e.g*., CV-1, NIH-3T3, CHO-Kl, COS-7). Such cell lines can be loaded with ⁸⁶Rb, an ion which can pass through potassium channels. The ⁸⁶Rb-loaded cells can be exposed to collections of substances (*e.g*., combinatorial libraries, natural products, analogues of lead compounds produced by medicinal chemistry) and those substances that are able to alter 86Rb efflux identified. Such substances are likely to be activators or inhibitors of calcium sensitive potassium channels containing human calcium sensitive potassium channel β2, β3a, β3b, β3c, or β3d subunit proteins.

Activators and inhibitors of calcium sensitive potassium channels containing human calcium sensitive potassium channel β2, β3a, β3b, β3c, or β3d subunit proteins are likely to be substances that are capable of binding to calcium sensitive potassium channels. Accordingly, one type of assay determines whether one or more of a collection of substances is capable of such binding.

Accordingly, the present invention provides a method for identifying substances that bind to calcium sensitive potassium channels containing human calcium sensitive potassium channel β2, β3a, β3b, β3c, or β3d subunit proteins comprising:
(a) providing cells expressing a calcium sensitive potassium channel containing human calcium sensitive potassium channel β2, β3a, β3b, β3c, or β3d subunit proteins;
(b) exposing the cells containing human calcium sensitive potassium channel β2, β3a, β3b, β3c, or β3d subunit proteins to a substance that is not known to bind calcium sensitive potassium channels;
(c) determining the amount of binding of the substance to the cells;
(d) comparing the amount of binding in step (c) to the amount of binding of the substance to control cells where the control cells are substantially identical to the cells of step (a) except that the control cells do not express human calcium sensitive potassium channel β2, β3a, β3b, β3c, or β3d subunit proteins;
where if the amount of binding in step (c) is greater than the amount of binding of the substance to control cells, then the substance binds to calcium sensitive potassium channels containing human calcium sensitive potassium channel β2, β3a, β3b, β3c, or β3d subunit proteins.

Another version of this assay makes use of compounds that are known to bind to calcium sensitive potassium channels containing human calcium sensitive potassium channel β2, β3a, β3b, β3c, or β3d subunit proteins. New binders are identified by virtue of their ability to potentiate, prevent, or displace the binding of the known compounds. Substances that have this ability are likely themselves to be inhibitors or activators of calcium sensitive potassium channels containing human calcium sensitive potassium channel β2, β3a, β3b, β3c, or β3d subunit proteins.

Accordingly, the present invention includes a method of identifying substances that bind calcium sensitive potassium channels containing human calcium sensitive potassium channel β2, β3a, β3b, β3c, or β3d subunit proteins and thus are likely to be inhibitors or activators of calcium sensitive potassium channels containing human calcium sensitive potassium channel β2, β3a, β3b, β3c, or β3d subunit proteins comprising:
(a) providing cells expressing calcium sensitive potassium channels containing human calcium sensitive potassium channel β2, β3a, β3b, β3c, or β3d subunit proteins;
(b) exposing the cells to a compound that is known to bind to the calcium sensitive potassium channels containing human calcium sensitive potassium channel β2, β3a, β3b, β3c, or β3d subunit proteins;
(c) determining the amount of binding of the compound to the cells in the presence and in the absence of a substance not known to bind to calcium sensitive potassium channels containing human calcium sensitive potassium channel β2, β3a, β3b, β3c, or β3d subunit proteins;
where if the amount of binding of the compound in the presence of the substance differs from that in the absence of the substance, then the substance binds calcium sensitive potassium channels containing human calcium sensitive potassium channel β2, β3a, β3b, β3c, or β3d subunit proteins and is likely to be an inhibitor or activator of calcium sensitive potassium channels containing human calcium sensitive potassium channel β2, β3a, β3b, β3c, or β3d subunit proteins.

Generally, the known compound is labeled (*e.g*., radioactively, enzymatically, fluorescently) in order to facilitate measuring its binding to the calcium sensitive potassium channels.

Once a substance has been identified by the above-described methods, it can be assayed in functional tests, such as those described herein, in order to determine whether it is an inhibitor or an activator.

In particular embodiments, the compound known to bind calcium sensitive potassium channels containing human calcium sensitive potassium channel β2, β3a, β3b, β3c, or β3d subunit proteins is selected from the group consisting of: charybdotoxin, iberiotoxin, and dehydrosoyasaponin.

The present invention includes a method of identifying activators or inhibitors of calcium sensitive potassium channels containing human calcium sensitive potassium channel β2, β3a, β3b, β3c, or β3d subunit proteins comprising:
(a) recombinantly expressing human calcium sensitive potassium channel β2, β3a, β3b, β3c, or β3d subunit proteins or mutant human calcium sensitive potassium channel β2, β3a, β3b, β3c, or β3d subunit proteins in a host cell so that the recombinantly expressed human calcium sensitive potassium channel β2, β3a, β3b, β3c, or β3d subunit proteins form calcium sensitive potassium channels by forming heteromers with other calcium sensitive potassium channel subunit proteins:
(b) measuring the biological activity of the calcium sensitive potassium channels formed in step (a) in the presence and in the absence of a substance suspected of being an activator or an inhibitor of calcium sensitive potassium channels containing human calcium sensitive potassium channel β2, β3a, β3b, β3c, or β3d subunit proteins;
where a change in the biological activity of the calcium sensitive potassium channels formed in step (a) in the presence as compared to the absence of the substance indicates that the substance is an activator or an inhibitor of calcium sensitive potassium channels containing human calcium sensitive potassium channel β2, β3a, β3b, β3c, or β3d subunit proteins.

It may be advantageous to recombinantly express other subunits of calcium sensitive potassium channels such as, *e.g*., an α subunit. Alternatively, it may be advantageous to use host cells that endogenously express such other subunits.

In particular embodiments, the biological activity is the production of a calcium sensitive potassium current, a FRET signal, or the efflux of ⁸⁶Rb.

In particular embodiments, a vector encoding human calcium sensitive potassium channel β2, β3a, β3b, β3c, or β3d subunit proteins is transferred into *Xenopus* oocytes in order to cause the expression of human calcium sensitive potassium channel β2, β3a, β3b, β3c, or β3d subunit proteins in the oocytes. Alternatively, RNA encoding human calcium sensitive potassium channel β2, β3a, β3b, β3c, or β3d subunit proteins can be prepared *in vitro* and injected into the oocytes, also resulting in the expression of human calcium sensitive potassium channel β2, β3a, β3b, β3c, or β3d subunit proteins in the oocytes. Following expression of the human calcium sensitive potassium channel β2, β3a, β3b, β3c, or β3d subunit proteins in the oocytes, and following the formation of calcium sensitive potassium channels containing these subunits and other calcium sensitive potassium channel subunits (which other subunits may also be transferred into the oocytes), membrane currents are measured after the transmembrane voltage and/or internal calcium concentration is changed in steps. A change in membrane current is observed when the calcium sensitive potassium channels open or close, allowing or inhibiting potassium ion flow, respectively. Similar oocyte studies were reported for KCNQ2 and KCNQ3 potassium channels in Wang et al., 1998, Science 282:1890-1893 and this reference and references cited therein can be consulted for guidance as to how to carry out such studies.

Inhibitors or activators of calcium sensitive potassium channels containing human calcium sensitive potassium channel β2, β3a, β3b, β3c, or β3d subunit proteins can be identified by exposing the oocytes to collections of substances and determining whether the substances can block or diminish, or activate or enhance the membrane currents observed in the absence of the substance.

Accordingly, the present invention provides a method of identifying inhibitors or activators of calcium sensitive potassium channels containing human calcium sensitive potassium channel β2, β3a, β3b, β3c, or β3d subunit proteins comprising:
(a) expressing human calcium sensitive potassium channel β2, β3a, β3b, β3c, or β3d subunit proteins in a heterologous system such that calcium sensitive potassium channels containing the human calcium sensitive potassium channel β2, β3a, β3b, β3c, or β3d subunit proteins are formed;
(b) changing the transmembrane potential or internal calcium concentration of the heterologous system in the presence and the absence of a substance suspected of being an inhibitor or activator of calcium sensitive potassium channels containing human calcium sensitive potassium channel β2, β3a, β3b, β3c, or β3d subunit proteins;
(c) measuring membrane potassium currents following step (b);
where if the membrane potassium currents measured in step (c) are greater in the absence rather than in the presence of the substance, then the substance is an inhibitor of calcium sensitive potassium channels containing human calcium sensitive potassium channel β2, β3a, β3b, β3c, or β3d subunit proteins;
where if the membrane potassium currents measured in step (c) are less in the absence rather than in the presence of the substance, then the substance is an activator of calcium sensitive potassium channels containing human calcium sensitive potassium channel β2, β3a, β3b, β3c, or β3d subunit proteins.

In particular embodiments, the heterologous system is selected from the group consisting of: *Xenopus* oocytes and a mammalian cell line.

The present invention also includes assays for the identification of activators and inhibitors of calcium sensitive potassium channels containing human calcium sensitive potassium channel β2, β3a, β3b, β3c, or β3d subunit proteins that are based upon fluorescence resonance energy transfer (FRET) between a first and a second fluorescent dye where the first dye is bound to one side of the plasma membrane of a cell expressing calcium sensitive potassium channels containing human calcium sensitive potassium channel β2, β3a, β3b, β3c, or β3d subunit proteins and the second dye is free to shuttle from one face of the membrane to the other face in response to changes in membrane potential. In certain embodiments, the first dye is impenetrable to the plasma membrane of the cells and is bound predominately to the extracellular surface of the plasma membrane. The second dye is trapped within the plasma membrane but is free to diffuse within the membrane. At normal (*i.e*., negative) resting potentials of the membrane, the second dye is bound predominately to the inner surface of the extracellular face of the plasma membrane, thus placing the second dye in close proximity to the first dye. This close proximity allows for the generation of a large amount of FRET between the two dyes. Following membrane depolarization, the second dye moves from the extracellular face of the membrane to the intracellular face, thus increasing the distance between the dyes. This increased distance results in a decrease in FRET, with a corresponding increase in fluorescent emission derived from the first dye and a corresponding decrease in the fluorescent emission from the second dye. In this way, the amount of FRET between the two dyes can be used to measure the polarization state of the membrane. For a fuller description of this technique, see González & Tsien, 1997, Chemistry & Biology 4:269-277. See also González & Tsien, 1995, Biophys. J. 69:1272-1280 and U.S. Patent No. 5,661,035.

In certain embodiments, the first dye is a fluorescent lectin or a fluorescent phospholipid that acts as the fluorescent donor. Examples of such a first dye are: a coumarin-labeled phosphatidylethanolamine (*e.g*., N-(6-chloro-7-hydroxy-2-oxo-2H-1-benzopyran-3-carboxamidoacetyl)-dimyristoylphosphatidylethanolamine) or N-(7-nitrobenz-2-oxa-1,3-diazol-4-yl)-dipalmitoylphosphatidylethanolamine); a fluorescently-labeled lectin (*e.g.*, fluorescein-labeled wheat germ agglutinin). In certain embodiments, the second dye is an oxonol that acts as the fluorescent acceptor. Examples of such a second dye are: bis(1,3-dialkyl-2-thiobarbiturate)trimethineoxonols (*e.g*., bis(1,3-dihexyl-2-thiobarbiturate)trimethineoxonol) or pentamethineoxonol analogues (*e.g.*, bis(1,3-dihexyl-2-thiobarbiturate)pentamethineoxonol; or bis(1,3-dibutyl-2-thiobarbiturate)pentamethineoxonol). See González & Tsien, 1997, Chemistry & Biology 4:269-277 for methods of synthesizing various dyes suitable for use in the present invention. In certain embodiments, the assay may comprise a natural carotenoid, *e.g*., astaxanthin, in order to reduce photodynamic damage due to singlet oxygen.

The above described assays can be utilized to discover activators and inhibitors of calcium sensitive potassium channels containing human calcium sensitive potassium channel β2, β3a, β3b, β3c, or β3d subunit proteins. Such assays will generally utilize cells that express calcium sensitive potassium channels containing human calcium sensitive potassium channel β2, β3a, β3b, β3c, or β3d subunit proteins, *e.g*., by transfection with expression vectors encoding human calcium sensitive potassium channel β2, β3a, β3b, β3c, or β3d subunit proteins and, optionally, other calcium sensitive potassium channel subunits. In such cells, depolarization of the membrane potential as well as increases in intracellular calcium concentration will tend to open the calcium sensitive potassium channels. This will result in potassium efflux, tending to counteract the depolarization. In other words, the cells will tend to repolarize. The presence of an inhibitor of the calcium sensitive potassium channel will prevent, or diminish, this repolarization. Thus, membrane potential will tend to become more positive (*i.e.*, depolarized) in the presence of inhibitors. Activators of the calcium sensitive potassium channel will open this channel and thus tend to hyperpolarize the membrane potential. Changes in membrane potential (depolarizations and hyperpolarizations) that are caused by inhibitors and activators of calcium sensitive potassium channels can be monitored by the assays using FRET described above.

Accordingly, the present invention provides a method of identifying activators of calcium sensitive potassium channels containing human calcium sensitive potassium channel β2, β3a, β3b, β3c, or β3d subunit proteins comprising:
(a) providing test cells comprising:
   (1) an expression vector that directs the expression of human calcium sensitive potassium channel β2, β3a, β3b, β3c, or β3d subunit proteins in the cells so that calcium sensitive potassium channels containing human β2, β3a, β3b, β3c, or β3d subunit proteins are formed in the cells;
   (2) a first fluorescent dye, where the first dye is bound to one side of the plasma membrane of the cells; and
   (3) a second fluorescent dye, where the second fluorescent dye is free to shuttle from one face of the plasma membrane of the cells to the other face in response to changes in membrane potential;
(b) exposing the test cells to a substance that is suspected of being an activator of calcium sensitive potassium channels containing human calcium sensitive potassium channel β2, β3a, β3b, β3c, or β3d subunit proteins;
(c) measuring the amount of fluorescence resonance energy transfer (FRET) in the test cells that have been exposed to the substance;
(d) comparing the amount of FRET exhibited by the test cells that have been exposed to the substance with the amount of FRET exhibited by control cells;
wherein if the amount of FRET exhibited by the test cells is greater than the amount of FRET exhibited by the control cells, the substance is an activator of calcium sensitive potassium channels containing human calcium sensitive potassium channel β2, β3a, β3b, β3c, or β3d,-subunit proteins:
where the control cells are either (1) cells that are essentially the same as the test cells except that they do not comprise at least one of the items listed at (a) (1)-(3) but have been exposed to the substance; or (2) test cells that have not been exposed to the substance.

The present invention also provides a method of identifying inhibitors of calcium sensitive potassium channels containing human calcium sensitive potassium channel β2, β3a, β3b, β3c, or β3d subunit proteins comprising:
(a) providing test cells comprising:
   (1) an expression vector that directs the expression of human calcium sensitive potassium channel β2, β3a, β3b, β3c, or β3d subunit proteins in the cells so that calcium sensitive potassium channels containing human β2, β3a, β3b, β3c, or β3d subunit proteins are formed in the cells;
   (2) a first fluorescent dye, where the first dye is bound to one side of the plasma membrane of the cells; and
   (3) a second fluorescent dye, where the second fluorescent dye is free to shuttle from one face of the plasma membrane of the cells to the other face in response to changes in membrane potential;
(b) exposing the test cells to a substance that is suspected of being an inhibitor of calcium sensitive potassium channels containing human calcium sensitive potassium channel β2, β3a, β3b, β3c, or β3d subunit proteins;
(c) measuring the amount of fluorescence resonance energy transfer (FRET) in the test cells that have been exposed to the substance;
(d) comparing the amount of FRET exhibited by the test cells that have been exposed to the substance with the amount of FRET exhibited by control cells;
wherein if the amount of FRET exhibited by the test cells is less than the amount of FRET exhibited by the control cells, the substance is an inhibitor of calcium sensitive potassium channels containing human calcium sensitive potassium channel β2, β3a, β3b, β3c, or β3d subunit proteins;
where the control cells are either (1) cells that are essentially the same as the test cells except that they do not comprise at least one of the items listed at (a) (1)-(3) but have been exposed to the substance; or (2) test cells that have not been exposed to the substance.

In a variation of the assay described above, instead of the cell's membrane potential being allowed to reach steady state on its own, the membrane potential is artificially set at a potential in which the calcium sensitive potassium channels containing human calcium sensitive potassium channel β2, β3a, β3b, β3c, or β3d subunit proteins are open. This can be done, *e.g.,* by variation of the external K⁺ concentration in a known manner (*e.g*., increased concentrations of external K⁺). If such cells, having open calcium sensitive potassium channels containing human calcium sensitive potassium channel β2, β3a, β3b, β3c, or β3d subunit proteins, are exposed to inhibitors, the calcium sensitive potassium channels will be blocked, and the cells' membrane potentials will be depolarized. This depolarization can be observed as a decrease in FRET.

In a variation of the assay described above, instead of the cell's membrane potential being allowed to reach steady state on its own, the membrane potential is artificially set at a potential in which the calcium sensitive potassium channels containing human calcium sensitive potassium channel β2, β3a, β3b, β3c, or β3d subunit proteins are open by coexpression of another depolarizing current. If such cells, having open calcium sensitive potassium channels containing human calcium sensitive potassium channel β2, β3a, β3b, β3c, or β3d subunit proteins, are exposed to inhibitors, the calcium sensitive potassium channels will be blocked, and the cells' membrane potentials will be depolarized. This depolarization can be observed as a decrease in FRET. If such cells, having open calcium sensitive potassium channels containing human calcium sensitive potassium channel β2, β3a, β3b, β3c, or β3d subunit proteins, are exposed to activators, the balance of the calcium sensitive potassium current and the additional depolarizing current will shift *(i.e.,* the calcium sensitive potassium current will make a larger contribution to the total current) and the cell's membrane potential will be hyperpolarized. This polarization may be observed as an increase in FRET.

Accordingly, the present invention provides a method of identifying inhibitors or activators of calcium sensitive potassium channels containing human calcium sensitive potassium channel β2, β3a, β3b, β3c, or β3d subunit proteins comprising:
(a) providing cells comprising:
   (1) an expression vector that directs the expression of human calcium sensitive potassium channel β2, β3a, β3b, β3c, or β3d subunit proteins in the cells so the calcium sensitive potassium channels containing human β2, β3a, β3b, β3c, or β3d subunit proteins are formed in the cells;
   (2) a first fluorescent dye, where the first dye is bound to one side of the plasma membrane of the cells; and
   (3) a second fluorescent dye, where the second fluorescent dye is free to shuttle from one face of the plasma membrane of the cells to the other face in response to changes in membrane potential;
(b) adjusting the membrane potential of the cells such that the ion channel formed by the calcium sensitive potassium channels containing human calcium sensitive potassium channel β2, β3a, β3b, β3c, or β3d subunit proteins is open;
(c) measuring the amount of fluorescence resonance energy transfer (FRET) in the test cells;
(d) repeating step (b) and step (c) while the cells are exposed to a substance that is suspected of being an inhibitor or activator of calcium sensitive potassium channels containing human calcium sensitive potassium channel β2, β3a, β3b, β3c, or β3d subunit proteins;
where if the amount of FRET exhibited by the cells that are exposed to the substance is different than the amount of FRET exhibited by the cells that have not been exposed to the substance, then the substance is an inhibitor or activator of calcium sensitive potassium channels containing human calcium sensitive potassium channel β2, β3a, β3b, β3c, or β3d subunit proteins.

In particular embodiments of the above-described methods, the cells contain an expression vector encoding a human calcium sensitive potassium channel β2, β3a, β3b, β3c, or β3d subunit protein. In particular embodiments of the above-described methods, the expression vector is transfected into the test cells.

In particular embodiments of the above-described methods, the human calcium sensitive potassium channel β2, β3a, β3b, β3c, or β3d subunit protein has an amino acid sequence selected from the group consisting of: SEQ.ID.NO.:2, 4, 6, 8, and 10.

In particular embodiments of the above-described methods, the first fluorescent dye is selected from the group consisting of: a fluorescent lectin; a fluorescent phospholipid; a coumarin-labeled phosphatidylethanolamine; N-(6-chloro-7-hydroxy-2-oxo-2H--1-benzopyran-3-carboxamidoacetyl)-dimyristoylphosphatidyl-ethanolamine); N-(7-nitrobenz-2-oxa-1,3,-diazol-4-yl)-dipalmitoyl-phosphatidylethanolamine); and fluorescein-labeled wheat germ agglutinin.

In particular embodiments of the above-described methods, the second fluorescent dye is selected from the group consisting of: an oxonol that acts as the fluorescent acceptor; bis(1,3-dialkyl-2-thiobarbiturate)trimethineoxonols; bis(1,3-dihexyl-2-thiobarbiturate)trimethineoxonol; bis(1,3-dialkyl-2-thiobarbiturate) quatramethineoxonols; bis(1,3-dialkyl-2-thiobarbiturate)pentamethineoxonols; bis(1,3-dihexyl-2-thiobarbiturate)pentamethineoxonol; bis(1,3-dibutyl-2-thiobarbiturate)pentamethineoxonol); and bis(1,3-dialkyl-2-thiobarbiturate) hexamethineoxonols.

In a particular embodiment of the above-described methods, the cells are eukaryotic cells. In another embodiment, the cells are mammalian cells. In other embodiments, the cells are L cells L-M(TK-) (ATCC CCL 1.3), L cells L-M (ATCC CCL 1.2), 293 (ATCC CRL 1573), Raji (ATCC CCL 86), CV-1 (ATCC CCL 70), COS-1 (ATCC CRL 1650), COS-7 (ATCC CRL 1651), CHO-KI (ATCC CCL 61), 3T3 (ATCC CCL 92), NIH/3T3 (ATCC CRL 1658), HeLa (ATCC CCL 2), C127I (ATCC CRL 1616), BS-C-1 (ATCC CCL 26), MRC-5 (ATCC CCL 171), *Xenopus* melanophores, or *Xenopus* oocytes.

In particular embodiments of the above-described methods, the control cells do not comprise item (a)(1) but do comprise items (a)(2) and (a)(3).

In assays to identify activators or inhibitors of calcium sensitive potassium channels containing human calcium sensitive potassium channel β2, β3a, β3b, β3c, or β3d subunit proteins, it may be advantageous to co-express another calcium sensitive potassium channel subunit besides the human calcium sensitive potassium channel β2, β3a, β3b, β3c, or β3d subunit. In particular, it may be advantageous to co-express a calcium sensitive potassium channel α subunit. Preferably, this is done by co-transfecting into the cells an expression vector encoding the other subunit. Suitable other subunits are, *e.g*., the human calcium sensitive potassium channel α subunit *h-slo* (GenBank accession no. U11058), the mouse calcium sensitive potassium channel α subunit *m-slo* (GenBank accession no. U09383), the small conductance calcium sensitive potassium α subunits (GenBank accession nos. U69883, U69882, AF031815), or the intermediate conductance calcium sensitive potassium channel α subunit (GenBank accession no. AF022797).

Small regions of genomic sequences in proximity to a gene often regulate the transcription of that gene. These sequences are referred to as cis-acting elements. The proteins that bind these DNA sequences and directly affect the ability of the transcriptional machinery to bind or transcribe the gene are referred to as transacting elements. The cis-acting transcriptional regulatory elements are most often 5' of the transcription start site, but have been located within and 3' of the transcribed portion of genes as well. Depending on their effects on the rate of transcription, these sequences can be divided into three categories: promoters, enhancers, and repressors. A promoter independently allows transcription of the gene, while an enhancer increases the rate of transcription but is not capable of inducing transcription independently of the promoter. A repressor element inhibits transcription directed by a promoter element. Methods for identifying these elements are well know in the field and are described in Ausubel et al., eds., 1989, Current Protocols in Molecular Biology, sections 9.6-9.8, and 12.0-12.11, John Wiley & Sons, New York, NY.

Accordingly, the novel genomic sequences (SEQ.ID.NO.:20, Figure 8) and isolated BAC clones of the present invention make possible methods for identifying 1) DNA sequences required for transcriptional control of gene expression, 2) proteins involved in transcriptional regulation and 3) compounds which modulate the rate of transcription of the β3 gene. Such assays utilize isolated and/or recombinant DNA comprising portions of SEQ.ID.NO.:20, positions 1 to 17,436, inserted into vectors upstream of the open reading frame of a reporter protein.

Useful reporter proteins are ones that are not expressed in the cells to be assayed (or are easily distinguished from endogenous proteins), have a linear relationship between the abundance of the transcript and the abundance of the reporter protein, and have a large window between the minimum detection level and saturation of detection system. Ideally, the abundance of the reporter protein is quickly measured by an enzymatic reaction, fluorescence detection, immunoassay or other means. Typical reporter proteins include, but are not limited to, the following: Chloramphenicol Acetyltransferase (CAT), firefly luciferase, Beta-Lactamase, Beta-Galactosidase, Secreted Alkaline Phosphatase (SEAP), human Growth Hormone (hGH), Green Fluorescent Protein (GFP) and GFP derivatives. Reporter vectors incorporating these proteins are commercially available, as are similar reporter vectors containing constitutive promoters, enhancers, or both (Clontech).

The present invention provides a method for identifying nucleotide sequences involved in transcriptional regulation of β3 gene expression. Once a fragment of at least 6 contiguous nucleotides of DNA from SEQ.ID.NO.:20, positions 1-17,436, has been inserted upstream of the reporter cDNA in a promoter-reporter vector, the vector is then transfected into cells that either do or do not endogenously express one or more of the calcium sensitive potassium channel subunits β3a, β3b, β3c or β3d. Promoter-reporter vectors may contain promoters, enhancers, both, or neither. Transfected cells are then assayed for the amount of reporter protein present. Because both transfection efficiency and transcription rate directly affect reporter protein level, it is useful in these assays to determine the transfection efficiency by co-transfecting a second vector (molar ratio 1:1) containing a distinct reporter behind a constitutive promoter, and determining the fraction of transfected cells.

In versions of the above assay, vectors are constructed with fragments of SEQ.ID.NO.:20 inserted upstream of a reporter cDNA with no other enhancer or promoter elements. These vectors (with and without fragments of SEQ.ID.NO.:20) are transfected into cells that endogenously express β3 subunits. Calcium sensitive potassium channel β3 subunit promoter elements are identified by the ability of these 5' gene fragments to stimulate reporter expression above the levels observed in the parent vector. The minimum required promoter sequence is then identified by successively deleting regions of the identified promoter fragment, and repeating the assay.

Another version of the assay incorporates fragments of SEQ.ID.NO.:20 inserted upstream of the reporter cDNA in a promoter-reporter vector containing an enhancer element. These vectors (with and without fragments of SEQ.ID.NO.:20) are transfected into cells that endogenously express β3 subunits. Weak calcium sensitive potassium channel β3 subunit promoter elements are identified by the ability of these 5' gene fragments to stimulate reporter expression above the levels observed in the parent vector. The minimum required weak promoter sequence can then be identified by successively deleting regions of the identified weak promoter fragment and repeating the assay

A different version of the assay incorporates fragments of SEQ.ID.NO.:20 inserted upstream of the reporter cDNA in a promoter-reporter vector with a constitutive promoter upstream. These vectors (with and without fragments of SEQ.ID.NO.:20) are transfected into cells that do not endogenously express β3 subunits. Calcium sensitive potassium channel β3 subunit repressor elements are identified by the ability of these 5' gene fragments to prevent or reduce reporter expression below the levels observed in the parent vector. The minimum required repressor sequence is then identified by successively deleting regions of the identified repressor fragment and repeating the assay.

In view of the above, the present invention provides a method of identifying DNA sequences in the β3 gene that promote, enhance, or repress gene transcription comprising:
(a) constructing a promoter-reporter vector such that fragments of the promoter region of the β3 gene (SEQ.ID.NO.:20, nucleotides 1 to 17,436) precede the coding cDNA sequence of a reporter gene which encodes a reporter protein;
(b) transfecting the vector into cells and measuring the abundance of the reporter protein encoded by the vector;
(c) comparing the abundance of the reporter protein in the cells of step (b) to the abundance of the reporter protein in cells transfected with the vector without fragments of the promoter region of the β3 gene;
where fragments of the promoter region of the β3 gene which increase the abundance of the reporter protein in the absence of other promoter elements only in cells which endogenously express β3a, β3b, β3c, or β3d subunits are promoter elements; sequences which decrease the abundance of the reporter protein in the presence of an unrelated constitutive promoter element in cells which do not endogenously express β3a, β3b, β3c, or β3d subunits are repressor elements; and sequences which increase the abundance of the reporter protein in the presence of an unrelated constitutive promoter element in cells which endogenously express β3a, β3b, β3c, or β3d subunits are enhancer elements.

In particular embodiments, the vector contains promoter or enhancer sequence elements which function independently of the fragments of the promoter region of the β3 gene.

In particular embodiments, the abundance of the reporter protein is normalized with respect to the fraction of transfected cells.

The binding of nuclear proteins to these sequences can be confirmed by gel-shift assays. A radiolabeled DNA fragment corresponding to the minimal sequence required to affect transcription is incubated with nuclear protein extracts from cells used to identify the regulatory DNA element, or tissues endogenously expressing β3 subunits. If a protein factor binds that sequence, the mobility in a gel will be altered, resulting in an apparent shift in the size of the radiolabeled fragment.

Transcription factors often are able to recognize more than one specific nucleotide sequence. As such, variations of sequences identified as minimal promoters, enhancers or repressors necessary for transcriptional regulation of the β3 gene in SEQ.ID.NO.:20, positions 1-17,436, which retain the ability to influence transcription as detected in the above described assays are intended to be included in the present invention.

Minimal promoter, enhancer or repressor DNA fragments thus identified can then be used to identify and/or isolate proteins that influence transcriptional activity of the β3 gene. Several methods are well known in the field, some of which are described in Ausubel et al., eds., 1989, Current Protocols in Molecular Biology, sections 12.0-12.11, John Wiley & Sons, New York, NY.

In one method, gel shift assays described above can be performed with cloned or purified known transcription factors, to determine if they are capable of binding sequences involved in transcriptional regulation. Alternatively, super-shift assays can be performed in which an antibody that recognizes a particular transcription factor is added to the transcription factor-DNA complex. If the antibody binds to the transcription factor, which in turn binds the radiolabeled DNA fragment, the mobility of the complex in a gel is further altered, resulting in a super-shift compared to the DNA alone. Using antibodies that recognize a specific transcription factor, or a class of transcription factors, allows identification of the factors involved in β3 gene regulation. Variations of sequences identified as minimal promoters, enhancers or repressors necessary for transcriptional regulation of the β3 gene in SEQ.ID.NO.:20, positions 1-17,436, which retain the ability to undergo gel shifts or super-shifts as described in the above assays are intended to be included in the present invention.

In view of the above, the present invention provides a method of identifying DNA sequences in the β3 gene that promote, enhance, or repress gene transcription comprising:
(a) incubating radiolabeled fragments of double stranded DNA corresponding to sequences found in the promoter region of the β3 gene (SEQ.ID.NO.:20, nucleotides 1 to 17,436) with nuclear extracts from cells and separating the incubation on a gel;
where fragments of double stranded DNA corresponding to sequences found in the promoter region of the β3 gene that migrate differently in a gel ('undergo a shift') after incubation with nuclear extracts from cells are DNA sequences which bind nuclear factors which promote, enhance or repress β3 gene expression.

In particular embodiments, the fragments of double stranded DNA corresponding to sequences found in the promoter region of the β3 gene are identified by the method of claim 18.

In particular embodiments, the cells express β3a, β3b, β3c, or β3d subunits.

In particular embodiments, the cells do not express β3a, β3b, β3c, or β3d subunits.

The present invention provides a method of identifying nuclear factors involved in β3 gene transcription regulation comprising:
(a) incubating radiolabeled fragments of double stranded DNA corresponding to sequences found in the promoter region of the β3 gene (SEQ.ID.NO.:20, nucleotides 1 to 17,436) with cloned or purified transcription factors and separating the incubation on a gel;
where factors which bind β3 gene promoter sequence elements will induce a shift in the migration of the radiolabeled DNA fragments, and are involved in β3 gene transcription regulation.

In particular embodiments, the fragments of double stranded DNA corresponding to sequences found in the promoter region of the β3 gene are identified by the methods of claim 18 or 21.

The present invention provides a method of identifying nuclear factors involved in β3 gene transcription regulation comprising:
(a) incubating radiolabeled fragments of double stranded DNA corresponding to sequences found in the promoter region of the β3 gene (SEQ.ID.NO.:20, nucleotides 1 to 17,436) with nuclear extracts from cells and separating the incubation on a gel;
(b) adding an antibody that specifically recognizes a single transcription factor or a family of transcription factors to the incubation of step (a), followed by separating the incubation on a gel:
where a super-shift in mobility of the double stranded DNA in step (b) as compared to step (a) indicates that a transcription factor recognized by the antibody binds the double stranded DNA.

In another method, the transcription factors that bind SEQ.ED.NO.:20, positions 1-17,436, and regulate transcription can be purified. DNA fragments corresponding to the minimal sequence required to affect transcription are covalently linked to a matrix (typically an agarose bead). This matrix is then incubated with nuclear extracts of cells that contain factors which bind the minimal element. The matrix is then washed free of non-specific proteins and the factor(s) are eluted with an excess of the DNA element, or by denaturation. Purified proteins can then be identified by immunoassay, protein sequencing, or other means.

Accordingly, the present invention provides a method of identifying nuclear factors involved in β3 gene transcription regulation comprising:
(a) attaching fragments of double stranded DNA corresponding to sequences found in the promoter region of the β3 gene (SEQ.ID.NO.:20, nucleotides 1 to 17,436) to a stable matrix;
(b) incubating nuclear extracts from cells with the matrix;
(c) washing non-binding proteins from the nuclear extract from the matrix;
(d) eluting bound proteins from the matrix with excess double stranded DNA corresponding to sequences found in the promoter region of the β3 gene;
where the eluted proteins from step (d) are nuclear factors involved in β3 gene transcription regulation.

In particular embodiments, the method further comprises separating the eluted proteins from step (d) on a gel and staining the gel to test for purity of the eluted proteins.

In particular embodiments, the method further comprises sequencing the proteins that have been separated on the gel.

In particular embodiments, the method further comprises immunological analysis of the proteins that have been separated on the gel with antibodies directed towards known transcription factors to identify the eluted proteins by western blot or immunoprecipitation.

In particular embodiments, the fragments of double stranded DNA corresponding to sequences found in the promoter region of the β3 gene are identified by the methods of claim 18 or 21.

In a different approach, cDNAs encoding the transcription factors that bind SEQ.ID.NO.:20; positions 1-17,436 can be cloned by several methods. In one version, the minimal DNA sequence is radiolabeled and used to screen an expression library made from tissues or cell lines that endogenously express the β3 gene. Phage containing cDNA encoding the transcription factor are induced to express fusion proteins that target the transcription factor to its surface. Such phage plaques are identified by their ability to bind radiolabeled DNA sequences containing the minimal DNA sequence.

Accordingly, the present invention provides a method of identifying clones encoding nuclear factors involved in β3 gene transcription regulation by cloning comprising:
(a) screening an expression library with radiolabeled fragments of double stranded DNA corresponding to sequences found in the promoter region of the β3 gene (SEQ.ID.NO.:20, nucleotides 1 to 17,436)
(b) determining which clones of the library bind the radiolabeled fragments of double stranded DNA;
(c) amplifying and sequencing the clones of step (b).

In particular embodiments, the fragments of double stranded DNA corresponding to sequences found in the promoter region of the β3 gene are identified by the methods of claim 18 or 21.

Another cloning approach involves phage expressing transcription factor fusion proteins at their surface. In this approach, the minimal DNA sequence is linked to a matrix. A phage expression library is then passed over the matrix and washed. Only phage containing the transcription factor bind the matrix. Bound phage are eluted with excess minimal DNA sequence and purified. cDNA encoding the transcription factor is then isolated from the phage and sequenced.

Accordingly, the present invention provides a method of identifying nuclear factors involved in β3 gene transcription regulation by cloning comprising:
(a) attaching fragments of double stranded DNA corresponding to sequences found in the promoter region of the β3 gene (SEQ.ID.NO.:20, nucleotides 1 to 17,436) to a stable matrix;
(b) incubating phage expressing cDNA encoded fusion proteins at their surface with the matrix;
(c) removing phage that do not bind to the matrix by washing;
(d) eluting phage bound to the matrix with excess fragments of double stranded DNA corresponding to sequences found in the promoter region of the β3 gene;
where the phage eluted in step (d) encode nuclear factors involved in β3 gene transcription regulation.

In particular embodiments, the DNA corresponding to sequences found in the promoter region of the β3 gene are identified by the methods of claim 18 or 21.

In particular embodiments, the phage eluted at step (d) are amplified and sequenced.

A separate transcription factor cloning approach is the yeast 'one-hybrid' method (available in kit form from Clontech). In this method, yeast strains are made that contain several copies (three suggested) of the minimal element upstream of a reporter. A cDNA library is made such that each vector contains a cDNA that will be expressed as a fusion protein with the transcription activation domain of a yeast promoter. Thus, any fusion protein that specifically binds the DNA of interest will induce expression of the reporter protein. The vector containing the cDNA is then isolated from the yeast and sequenced.

Accordingly, the present invention provides a method of identifying nuclear factors involved in β3 gene transcription regulation by cloning comprising:
(a) constructing a yeast strain that contains a few to several copies of a fragment of double stranded DNA corresponding to sequences found in the promoter region of the β3 gene (SEQ.ID.NO.:20, nucleotides 1 to 17,436) preceding a cDNA encoding a reporter protein;
(b) constructing a cDNA library from cells in a vector that allows formation of fusion proteins encoded by the inserted cDNA and a transcription activation domain;
(c) transforming the library of (b) into the yeast strain of (a) and isolating colonies of yeast displaying expression of the reporter protein.

In particular embodiments, the fragments of double stranded DNA corresponding to sequences found in the promoter region of the β3 gene are identified by the methods of claim 18 or 21.

In particular embodiments, the method further comprises purifying the vectors from the isolated colonies and sequencing the cDNA in the vectors.

Since transcription factors often are able to recognize more than one specific nucleotide sequence, variations of sequences identified as minimal promoters, enhancers or repressors necessary for transcriptional regulation of the β3 gene in SEQ.ID.NO.:20; positions 1-17,436, that can be bound by transcription factors as detected in the above described assays are intended to be included in the present invention.

Identification of nucleotide sequences involved in transcriptional regulation of β3 gene expression by the methods described above allows for the development of assays that can be used to screen collections of substances to identify those substances that enhance or inhibit transcription of the β3 gene. Fragments of the promoter region of the β3 gene (SEQ.ID.NO.:20, nucleotides 1 to 17,436) that have been shown to be involved in transcriptional regulation are linked to the coding sequence of a reporter gene in a suitable vector and are then transferred to appropriate cells. The abundance of the reporter protein in the cells is determined. The cells are then exposed to compounds that are suspected of being capable of enhancing or inhibiting the rate of transcription of the β3 gene. If the compound actually is capable of enhancing the rate of transcription of the β3 gene, then the abundance of the reporter protein will be increased when the cells are exposed to the compound. Conversely, if the compound actually is capable of inhibiting the rate of transcription of the β3 gene, then the abundance of the reporter protein will be decreased when the cells are exposed to the compound.

Accordingly, the present invention provides a method of identifying substances that enhance or inhibit the rate of transcription of the β3 gene comprising:
(a) constructing a promoter-reporter vector such that fragments of the promoter region of the β3 gene (SEQ.ID.NO.:20, nucleotides 1 to 17,436) precede the coding cDNA sequence of a reporter gene which encodes a reporter protein;
(b) transfecting the vector into cells and measuring the abundance of the reporter protein encoded by the vector in the presence and absence of a compound;
where (1) if the presence of the compound decreases the abundance of the reporter protein, then the compound is a substance that inhibits the rate of transcription of the β3 gene; (2) if the presence of the compound increases the abundance of the reporter protein, then the compound is a substance that enhances the rate of transcription of the β3 gene.

In particular embodiments, the method further comprises a control in which the effect of the compound on the abundance of the reporter protein in control cells is measured, where the control cells are cells that are essentially the same as the cells of step (b) except that the control cells have been transfected with a vector that lacks fragments of the promoter region of the β3 gene.

While the above-described methods are explicitly directed to testing whether "a" substance is an activator or inhibitor of the transcription the β3 gene or the function of calcium sensitive potassium channels containing human calcium sensitive potassium channel β2, β3a, β3b, β3c, or β3d subunit proteins, it will be clear to one skilled in the art that such methods can be adapted to test collections of substances, *e.g.,* combinatorial libraries, to determine whether any members of such collections are activators or inhibitors of calcium sensitive potassium channels containing human calcium sensitive potassium channel β2, β3a, β3b, β3c, or β3d subunit proteins. Accordingly, the use of collections of substances, or individual members of such collections, as the substance in the above-described methods is within the scope of the present invention. In particular, it is envisioned that libraries that have been designed to incorporate chemical structures that are known to be associated with potassium ion channel modulation, *e.g*., dihydrobenzopyran libraries for potassium channel activators (International Patent Publication WO 95/30642) or biphenyl-derivative libraries for potassium channel inhibitors (International Patent Publication WO 95/04277) will be especially suitable.

The present invention includes pharmaceutical compositions comprising activators or inhibitors of human calcium sensitive potassium channel β2, β3a, β3b, β3c, or β3d subunit proteins that have been identified by the herein-described methods as well as activators or inhibitors of β3 gene transcription. The activators or inhibitors are generally combined with pharmaceutically acceptable carriers to form pharmaceutical compositions. Examples of such carriers and methods of formulation of pharmaceutical compositions containing activators or inhibitors and carriers can be found in Remington's Pharmaceutical Sciences. To form a pharmaceutically acceptable composition suitable for effective administration, such compositions will contain a therapeutically effective amount of the activators or inhibitors.

Therapeutic or prophylactic compositions are administered to an individual in amounts sufficient to treat or prevent conditions where human calcium sensitive potassium channel β2, β3a, β3b, β3c, or β3d subunit protein activity is abnormal. The effective amount can vary according to a variety of factors such as the individual's condition, weight, gender, and age. Other factors include the mode of administration. The appropriate amount can be determined by a skilled physician. Generally, an effective amount will be from about 0.01 to about 1,000, preferably from about 0.1 to about 250 and even more preferably from about 1 to about 50 mg per adult human per day.

Compositions can be used alone at appropriate dosages. Alternatively, co-administration or sequential administration of other agents can be desirable.

The compositions can be administered in a wide variety of therapeutic dosage forms in conventional vehicles for administration. For example, the compositions can be administered in such oral dosage forms as tablets, capsules (each including timed release and sustained release formulations), pills, powders, granules, elixirs, tinctures, solutions, suspensions, syrups and emulsions, or by injection. Likewise, they can also be administered in intravenous (both bolus and infusion), intraperitoneal, subcutaneous, topical with or without occlusion, or intramuscular form, all using forms well known to those of ordinary skill in the pharmaceutical arts.

Compositions can be administered in a single daily dose, or the total daily dosage can be administered in divided doses of two, three, four or more times daily. Furthermore, compositions can be administered in intranasal form via topical use of suitable intranasal vehicles, or via transdermal routes, using those forms of transdermal skin patches well known to those of ordinary skill in that art. To be administered in the form of a transdermal delivery system, the dosage administration will, of course, be continuous rather than intermittent throughout the dosage regimen.

The dosage regimen utilizing the compositions is selected in accordance with a variety of factors including type, species, age, weight, sex and medical condition of the patient; the severity of the condition to be treated; the route of administration; the renal, hepatic and cardiovascular function of the patient; and the particular composition thereof employed. A physician of ordinary skill can readily determine and prescribe the effective amount of the composition required to prevent, counter or arrest the progress of the condition. Optimal precision in achieving concentrations of composition within the range that yields efficacy without toxicity requires a regimen based on the kinetics of the composition's availability to target sites. This involves a consideration of the distribution, equilibrium, and elimination of a composition.

The inhibitors and activators of calcium sensitive potassium channels containing human calcium sensitive potassium channel β2, β3a, β3b, β3c, or β3d subunit proteins, or inhibitors and activators of β3 subunit transcription will be useful for treating a variety of diseases involving excessive or insufficient calcium sensitive potassium channel activity. Accordingly, the present invention includes a method of treating asthma, diabetes, glaucoma, pregnant human myometrium, cerebral ischemia, and conditions where stimulation of neurotransmitter release is desired such as Alzheimer's disease and stimulation of damaged nerves by administering to a patient a therapeutically effective amount of a substance that is an activator or an inhibitor of a calcium sensitive potassium channel containing a human calcium sensitive potassium channel β2, β3a, β3b, β3c, or β3d subunit protein, or an activator or an inhibitor of β3 subunit transcription.

The modulators of channel function or transcription activity of the present invention are also expected to be useful in conditions where currently marketed inhibitors of potassium channels such as glyburide, glipizide, and tolbutamide are useful, *e.g*., as antidiabetic agents. Calcium sensitive potassium channels contribute to the repolarization, and thus the de-excitation, of neurons. Thus, inhibitors of calcium sensitive potassium channels are expected to act as agents that tend to keep neurons in a depolarized, excited state. Many diseases, such as depression and memory disorders are thought to result from the impairment of neurotransmitter release. As agents that contribute to neuronal excitability, the inhibitors of the present invention are expected to useful in the treatment of such diseases since they will contribute to neuronal excitation and thus stimulate the release of neurotransmitters.

The activators of the present invention should be useful in conditions where it is desirable to decrease neuronal activity. Such conditions include, *e.g*., excessive smooth muscle tone, angina, asthma, hypertension, incontinence, pre-term labor, migraine, cerebral ischemia, and Irritabie Bowel Syndrome.

The calcium sensitive potassium channel subunits of the present invention are useful in conjunction with screens designed to identify activators and inhibitors of other ion channels. When screening compounds in order to identify potential pharmaceuticals that specifically interact with a target ion channel, it is necessary to ensure that the compounds identified are as specific as possible for the target ion channel. To do this, it is necessary to screen the compounds against as wide an array as possible of ion channels that are similar to the target ion channel. Thus, in order to find compounds that are potential pharmaceuticals that interact with ion channel A, it is not enough to ensure that the compounds interact with ion channel A (the "plus target") and produce the desired pharmacological effect through ion channel A. It is also necessary to determine that the compounds do not interact with ion channels B, C, D, *etc.* (the "minus targets"). In general, as part of a screening program, it is important to have as many minus targets as possible (see Hodgson, 1992, Bio/Technology 10:973-980, at 980). Human calcium sensitive potassium channel β2, β3a, β3b, β3c, or β3d subunit proteins, DNA encoding human calcium sensitive potassium channel β2, β3a, β3b, β3c, or β3d subunit proteins, and recombinant cells that have been engineered to express human calcium sensitive potassium channel β2, β3a, β3b, β3c, or β3d subunit proteins have utility in that they can be used as "minus targets" in screens designed to identify compounds that specifically interact with other ion channels. For example, Wang et al., 1998, Science 282:1890-1893 have shown that KCNQ2 and KCNQ3 form a heteromeric potassium ion channel know as the "M-channel." The M-channel is an important target for drug discovery since mutations in KCNQ2 and KCNQ3 are responsible for causing epilepsy (Biervert et al., 1998, Science 279:403-406; Singh et al., 1998, Nature Genet. 18:25-29; Schroeder et al., Nature 1998, 396:687-690). A screening program designed to identify activators or inhibitors of the M-channel would benefit greatly by the use of potassium channels comprising human calcium sensitive potassium channel β2, β3a, β3b, β3c, or β3d subunit proteins as minus targets.

The present invention also includes antibodies to the human calcium sensitive potassium channel β2, β3a, β3b, β3c, or β3d subunit proteins. Such antibodies may be polyclonal antibodies or monoclonal antibodies. The antibodies of the present invention can be raised against the entire human calcium sensitive potassium channel β2, β3a, β3b, β3c, or β3d subunit proteins or against suitable antigenic fragments of the subunit proteins that are coupled to suitable carriers, *e.g*., serum albumin or keyhole limpet hemocyanin, by methods well known in the art. Methods of identifying suitable antigenic fragments of a protein are known in the art. See, *e.g.,* Hopp & Woods, 1981, Proc. Natl. Acad. Sci. USA 78:3824-3828; and Jameson & Wolf, 1988, CABIOS (Computer Applications in the Biosciences) 4:181-186.

For the production of polyclonal antibodies, human calcium sensitive potassium channel β2, β3a, β3b, β3c, or β3d subunit proteins or antigenic fragments, coupled to a suitable carrier, are injected on a periodic basis into an appropriate non-human host animal such as, *e.g.*, rabbits, sheep, goats, rats, mice or chickens. The animals are bled periodically (or eggs collected) and sera obtained are tested for the presence of antibodies to the injected subunit or antigen. The injections can be intramuscular, intraperitoneal, subcutaneous, and the like, and can be accompanied with adjuvant.

For the production of monoclonal antibodies, human calcium sensitive potassium channel β2, β3a, β3b, β3c, or β3d subunit proteins or antigenic fragments, coupled to a suitable carrier, are injected into an appropriate non-human host animal as above for the production of polyclonal antibodies. In the case of monoclonal antibodies, the animal is generally a mouse. The animal's spleen cells are then immortalized, often by fusion with a myeloma cell, as described in Kohler & Milstein, 1975, Nature 256:495-497. For a fuller description of the production of monoclonal antibodies, see Antibodies: A Laboratory Manual, Harlow & Lane, eds., Cold Spring Harbor Laboratory Press, 1988.

Gene therapy may be used to introduce human calcium sensitive potassium channel β2, β3a, β3b, β3c, or β3d subunit proteins into the cells of target organs. Nucleotides encoding human calcium sensitive potassium channel β2, β3a, β3b, β3c, or β3d subunit proteins can be ligated into viral vectors which mediate transfer of the nucleotides by infection of recipient cells. Suitable viral vectors include retrovirus, adenovirus, adeno-associated virus, herpes virus, vaccinia virus, lentivirus, and polio virus based vectors. Alternatively, nucleotides encoding human calcium sensitive potassium channel β2, β3a, β3b, β3c, or β3d subunit proteins can be transferred into cells for gene therapy by non-viral techniques including receptor-mediated targeted transfer using ligand-nucleotide conjugates, lipofection, membrane fusion, or direct microinjection. These procedures and variations thereof are suitable for *ex vivo* as well as *in vivo* gene therapy. Gene therapy with human calcium sensitive potassium channel β2, β3a, β3b, β3c, or β3d subunit proteins will be particularly useful for the treatment of diseases where it is beneficial to elevate calcium sensitive potassium channel activity. cDNAs encoding mutant calcium sensitive potassium channel subunits, that display a dominant negative phenotype, may be particularly useful for gene therapy treatment of diseases where it is beneficial to decrease calcium sensitive potassium channel activity.

The present invention includes processes for cloning orthologues of human calcium sensitive potassium channel β2, β3a, β3b, β3c, or β3d subunits from non-human species. In general, such processes include preparing a PCR primer or a hybridization probe based upon SEQ.ID.NO.:1, 3, 5, 7, 9, or 20 that can be used to amplify a fragment containing the non-human calcium sensitive potassium channel β2, β3a, β3b, β3c, or β3d subunit (in the case of PCR) from a suitable DNA preparation or to select a cDNA or genomic clone containing the non-human calcium sensitive potassium channel β2, β3a, β3b, β3c, or β3d subunit from a suitable library. A preferred embodiment of this process is a process for cloning the calcium sensitive potassium channel β2, β3a, β3b, β3c, or β3d subunit from mouse.

By providing DNA encoding mouse calcium sensitive potassium channel β2, β3a, β3b, β3c, or β3d subunits, the present invention allows for the generation of an animal model of human diseases in which calcium sensitive potassium channel β2, β3a, β3b, β3c, or β3d subunit activity is abnormal. Such animal models can be generated by making transgenic "knockout" or "knockin" mice containing altered calcium sensitive potassium channel β2, β3a, β3b, β3c, or β3d subunit genes. Knockout mice can be generated in which portions of the mouse calcium sensitive potassium channel β2, β3a, β3b, β3c, or β3d subunit gene have been deleted. Knockin mice can be generated in which mutations that have been shown to lead to human disease are introduced into the mouse gene. Such knockout and knockin mice will be valuable tools in the study of the relationship between calcium sensitive potassium channels and disease and will provide important model systems in which to test potential pharmaceuticals or treatments for human diseases involving calcium sensitive potassium channels.

Accordingly, the present invention includes a method of producing a transgenic mouse comprising:
(a) designing PCR primers or an oligonucleotide probe based upon SEQ.ID.NO.:1, 3, 5, 7, 9 or 20 for use in cloning the mouse calcium sensitive potassium channel β2, β3a, β3b, β3c, or β3d subunit gene or cDNA;
(b) using the PCR primers or the oligonucleotide probe to clone at least a portion of the mouse calcium sensitive potassium channel β2, β3a, β3b, β3c, or β3d subunit gene or cDNA, the portion being large enough to use in making a transgenic mouse;
(c) producing a transgenic mouse having at least one copy of the mouse calcium sensitive potassium channel β2, β3a, β3b, β3c, or β3d subunit gene altered from its native state.

Methods of producing knockout and knockin mice are well known in the art. One method involves the use of gene-targeted ES cells in the generation of gene-targeted transgenic knockout mice and is described in, *e.g*., Thomas et al., 1987, Cell 51:503-512, and is reviewed elsewhere (Frohman et al., 1989, Cell 56:145-147; Capecchi, 1989, Trends in Genet. 5:70-76; Baribault et al., 1989, Mol. Biol. Med. 6:481-492).

Techniques are available to inactivate or alter any genetic region to virtually any mutation desired by using targeted homologous recombination to insert specific changes into chromosomal genes. Generally, use is made of a "targeting vector," *i.e*., a plasmid containing part of the genetic region it is desired to mutate. By virtue of the homology between this part of the genetic region on the plasmid and the corresponding genetic region on the chromosome, homologous recombination can be used to insert the plasmid into the genetic region, thus disrupting the genetic region. Usually, the targeting vector contains a selectable marker gene as well.

In comparison with homologous extrachromosomal recombination, which occurs at frequencies approaching 100%, homologous plasmid-chromosome recombination was originally reported to only be detected at frequencies between 10⁻⁶ and 10-3 (Lin et al., 1985, Proc. Natl. Acad. Sci. USA 82:1391-1395; Smithies et al., 1985, Nature 317: 230-234; Thomas et al., 1986, Cell 44:419-428). Nonhomologous plasmid-chromosome interactions are more frequent, occurring at levels 10⁵-fold (Lin et al., 1985, Proc. Natl. Acad. Sci. USA 82:1391-1395) to 10²-fold (Thomas et al., 1986, Cell 44:419-428) greater than comparable homologous insertion.

To overcome this low proportion of targeted recombination in murine ES cells, various strategies have been developed to detect or select rare homologous recombinants. One approach for detecting homologous alteration events uses the polymerase chain reaction (PCR) to screen pools of transformant cells for homologous insertion, followed by screening individual clones (Kim et al., 1988, Nucleic Acids Res. 16:8887-8903; Kim et al., 1991, Gene 103:227-233). Alternatively, a positive genetic selection approach has been developed in which a marker gene is constructed which will only be active if homologous insertion occurs, allowing these recombinants to be selected directly (Sedivy et al., 1989, Proc. Natl. Acad. Sci. USA 86:227-231). One of the most powerful approaches developed for selecting homologous recombinants is the positive-negative selection (PNS) method developed for genes for which no direct selection of the alteration exists (Mansour et al., 1988, Nature 336:348-352; Capecchi, 1989, Science 244:1288-1292; Capecchi, 1989, Trends in Genet. 5:70-76). The PNS method is more efficient for targeting genes which are not expressed at high levels because the marker gene has its own promoter. Nonhomologous recombinants are selected against by using the Herpes Simplex virus thymidine kinase (HSV-TK) gene and selecting against its nonhomologous insertion with herpes drugs such as gancyclovir (GANC) or FIAU (1-(2-deoxy 2-fluoro-B-D-arabinofluranosyl)-5-iodouracil). By this counter-selection, the percentage of homologous recombinants in the surviving transformants can be increased.

Other methods of producing transgenic mice involve microinjecting the male pronuclei of fertilized eggs. Such methods are well known in the art.

The present invention includes a transgenic, non-human animal in which the animal's genome contains DNA encoding at least a portion of a human calcium sensitive potassium channel β2, β3a, β3b, β3c, or β3d subunit.

The following non-limiting examples are presented to better illustrate the invention.

### EXAMPLE 1

### Identification of the human calcium sensitive potassium channel β2, β3a, β3b, β3c, or β3d subunits and cDNA cloning

DNA sequence encoding the β1 subunit was used to search the GenBank database for homologous sequences encoding novel subunits. This search yielded an EST with similarity to β1 (AA904191). A cDNA encoding the EST was purchased (Genome Systems) and sequenced in both directions. Synthetic oligonucleotide primers (SEQ.ID.NOs.:12 and 13) were used to amplify the coding region and a small portion of the 3' untranslated region (UTR) of this gene (β2). The coding region was then subcloned into a modified vector (pSP64T) containing an expanded polylinker between the 5' and 3' translation enhancer sequences (MVpl(+)).

The sequence of β2 was then used to search the GenBank database for additional novel beta subunits. The sequences from identified EST's were then used to search the database again. Several EST's were obtained in this iterative approach: AA195381, AA236930, AA236968, AA279911, AA761761, AA934876, AA195511, AA917510. The alignment of these sequences suggested they encoded the C-terminal portion of a novel β subunit, here designated β3. Available cDNAs encoding these ESTs were purchased (Genome Systems) and sequenced in both directions. None of these clones encoded full length protein based on the lack of 5' in-frame stop codons and amino acid alignments only to the middle of the first transmembrane segments of β1 and β2.

Unique and conserved portions of the individual subunits were used separately to search the databases for genomic sequences encoding these transcripts. A single 180 kilobase fragment of unidentified genomic sequence was identified using β3a, β3b and β3c specific fragments (GenBank accession number AC007823, version 2). Later versions of this entry contained a 40.4 kilobase contiguous fragment that contained all three specific fragments in the following order β3a, β3b and β3c. β3c is contiguous with the 5' end of the core sequence. See Figure 8.

A synthetic oligo, 5'-TTT ACA TTG TTA GTT TGC AGA CAG G-3' (SEQ.ID.NO.:19), annealing 3' of the β3 stop codon was used in a 5' RACE reaction as described in Clontech's Marathon Ready Spleen cDNA kit (catalog # 7412-1). This reaction yielded multiple products of varying sizes. Several fragments separated by electrophoresis were extracted from gel slices and cloned. Three distinct subunits were identified (β3a, β3b and β3c) in this manner.

To ensure novel subunits were not overlooked, the unfractionated product of the PCR amplification reaction was cloned directly into a TA cloning vector (pCR2.1, Invitrogen), without any attempt to isolate specific fragments. Colonies were then screened using a probe derived from EST AA761761 by the 'colony filter hybridization protocol' as described in *Current Protocols in Molecular Biology,* sections 6.1.1 and 6.3.1. DNA was prepared from hybridizing colonies. cDNAs with restriction digest patterns distinct from the original clones were sequenced in both directions. The open reading frames were determined and amplified using synthetic oligonucleotide primers (SEQ.ID.NOs.:14 through 18), and subcloned into MVpl(+). One additional unique subunit was identified: β3d.

### EXAMPLE 2

### Analysis of expression of human calcium sensitive potassium channel β2, β3a, β3b, β3c, or β3d subunits

*Northern blot analysis:* Northern blots containing poly(A+)-RNA from human heart, brain, placenta, lung, liver, skeletal muscle, kidney, pancreas, spleen, thymus, prostate, testes, ovary, small intestine, colon, and peripheral blood leukocytes were purchased from Clontech, Palo Alto, CA. The blots were probed with ³²P-labeled, randomly primed cDNA probes from β2 (nucleotides 268 to 1080 of SEQ.ID.NO.:1), β3a (nucleotides 70 to 384 of SEQ.ID.NO.:3), β3b (nucleotides 463 to 797 of SEQ.ID.NO.:5), and β3c/d (nucleotides 311 to 912 of SEQ.ID.NO.:7). The hybridization was carried out in 5X SSPE, 10X Denhardts solution, 50% Formamide, 2% SDS, 100ug/ml salmon sperm DNA at 42°C overnight. The washes were carried out stepwise in 2X SSC, 0.05% SDS at 42°C for 40 minutes, followed by 1X SSC, 0.05% SDS at 50°C for 40 minutes. High stringency washes were carried out at 0.1SSC, 0.05% SDS at 65°C for 40 minutes. Hybridization was detected either by exposure of the washed blots to X-ray film or by electronic detection using a phosphorimager.

*Electrophysiological analysis:* cRNAs were synthesized in vitro from plasmids encoding human *Slowpoke* α or the β2, β3a, β3b, β3c, or β3d subunits and injected into *Xenopus* oocytes (1.5 ng/oocyte of α subunit RNA +/- β subunit RNA at 1, 5, or 10X molar excess). Calcium sensitive potassium currents were recorded in inside-out patches. Recordings were performed under ionic conditions of symmetrical potassium. The standard pipette and bath solutions contained 116 mM potassium gluconate, 4 mM potassium chloride, 10 mM HEPES, pH 7.2. CaCl₂ was added to the bath solution to give final concentrations of free ionized calcium of 3-30 µM, taking into account the stability constant for calcium gluconate (15.9 M⁻¹). Currents were recorded using an EPC-7 amplifier (HEKA). The pClamp6.0 program (Axon Instruments) was used to generate voltage-clamp commands for data acquisition, and for analysis. NPₒ - voltage relations were determined at 3, 10 and 30 µM bath calcium using two methods: (1) calculation of macroscopic conductance from peak or steady-state currents at test potentials (-80 to 80 mV), or (2) measurement or calculation of tail currents peaks (-80 mV) at test potentials. Boltzmann functions were fit to the data and used to derive the half-maximal activation parameter (V_{1/2}). Maximal inactivation parameters (30 µM Ca²⁺ and 80 mV) were calculated from current traces or averaged current traces. Inactivation rates were determined from single exponential fits. Fractional non-inactivating current was calculated as steady-state/peak current; fractional inactivating current was estimated as peak current minus steady-state current divided by peak current.

The present invention is not to be limited in scope by the specific embodiments described herein. Indeed, various modifications of the invention in addition to those described herein will become apparent to those skilled in the art from the foregoing description. Such modifications are intended to fall within the scope of the appended claims.

Various publications are cited herein, the disclosures of which are incorporated by reference in their entireties.

## Claims

1. An isolated DNA comprising nucleotides encoding a human calcium sensitive potassium channel β3a, β3b, β3c, or β3d subunit protein.

2. The DNA of claim 1 comprising nucleotides encoding a polypeptide having an amino acid sequence selected from the group consisting of: SEQ.ID.NO.:4; SEQ.ID.NO.:4 with an asparagine at position 163 instead of a serine; SEQ.ID.NO.:6; SEQ.ID.NO.:6 with a serine at position 143 instead of an asparagine; SEQ.ID.NO.:8; SEQ.ID.NO.:8 with an asparagine at position 161 instead of a serine; SEQ.ID.NO.:10; SEQ.ID.NO.:10 with a serine at position 165 instead of an asparagine;and positions 2-246 of SEQ.ID.NO.:6.

3. The DNA of claim 1 comprising a nucleotide sequence selected from the group consisting of: SEQ.ID.NO.: 3, 5, 7, 9, and 20.

4. The DNA of claim 1 comprising a nucleotide sequence selected from the group consisting of: positions 341 to
1171 of SEQ.ID.NO.:3, positions 796 to 1566 of SEQ.ID.NO.:5, positions 869 to
1693 of SEQ.ID.NO.:7, and positions 457 to 1293 of SEQ.ID.NO.:9.

5. An isolated DNA that hybridizes under stringent conditions to the DNA of claim 2.

6. An expression vector comprising the DNA of claim 1.

7. A recombinant host cell comprising the DNA of claim 1.

8. An isolated human calcium sensitive potassium channel β3a, β3b, β3c, or β3d subunit protein.

9. The protein of claim 8 having an amino acid sequence selected from the group consisting of: SEQ.ID.NO.:4; SEQ.ID.NO.:4 with an asparagine at position 163 instead of a serine; SEQ.ID.NO.:6; SEQ-ID.NO.:6 with a serine at position 143 instead of an asparagine; SEQ.ID.NO.:8; SEQ.ID.NO.:8 with an asparagine at position 161 instead of a serine; SEQ.ID.NO.:10; SEQ.ID-NO.:10 with a serine at position 165 instead of an asparagine; and positions 2-246 of SEQ.ID.NO.:6.

10. The protein of claim 8 containing a single amino acid substitution.

11. The protein of claim 8 containing two or more amino acid substitutions where the amino acid substitutions do not occur in conserved positions.

12. A polypeptide having at least 80% sequence identity to the protein of claim 9 when measured by BLAST or FASTA.

13. An antibody that binds specifically to a human calcium sensitive potassium channel β3a, β3b, β3c, or β3d subunit protein; or that binds specifically to the β3 subunit family of proteins by binding to the conserved core.

14. A DNA or RNA oligonucleotide probe comprising at least 15 contiguous nucleotides of at least one of a sequence selected from the group consisting of: SEQ.)D.NO.:.3, 5, 7, 9, and 20.

15. A method for identifying substances that bind to calcium sensitive potassium channels containing human calcium sensitive potassium channel β3a, β3b, β3c, or β3d subunit proteins comprising:
(a) providing cells expressing a calcium sensitive potassium channel containing human calcium sensitive potassium channel β3a, β3b, β3c, or β3d subunit proteins;
(b) exposing the cells to a substance that is not known to bind calcium sensitive potassium channels containing human calcium sensitive potassium channel β3a, β3b, β3c, or β3d subunit proteins;
(c) determining the amount of binding of the substance to the cells;
(d) comparing the amount of binding in step (c) to the amount of binding of the substance to control cells where the control cells are substantially identical to the cells of step (a) except that the control cells do not express human calcium sensitive potassium channel β3a, β3b, β3c, or β3d subunit proteins;
where if the amount of binding in step (c) is greater than the amount of binding of the substance to control cells, then the substance binds to calcium sensitive potassium channels containing human calcium sensitive potassium channel β3a, β3b, β3c, or β3d subunit proteins.

16. A method of identifying substances that bind calcium sensitive potassium channels containing human calcium sensitive potassium channel β3a, β3b, β3c, or β3d subunit proteins and thus are likely to be inhibitors or activators of calcium sensitive potassium channels containing human calcium sensitive potassium channel β3a, β3b, β3c, or β3d subunit proteins comprising:
(a) providing cells expressing calcium sensitive potassium channels containing human calcium sensitive potassium channel β3a, β3b, β3c, or β3d subunit proteins;
(b) exposing the cells to a compound that is known to bind to the calcium sensitive potassium channels containing human calcium sensitive potassium channel β3a, β3b, β3c, or β3d subunit proteins;
(c) determining the amount of binding of the compound to the cells in the presence and in the absence of a substance not known to bind to calcium sensitive potassium channels containing human calcium sensitive potassium channel β3a, β3b, β3c, or β3d subunit proteins;
where if the amount of binding of the compound in the presence of the substance differs from that in the absence of the substance, then the substance binds calcium sensitive potassium channels containing human calcium sensitive potassium channel β3a, β3b, β3c, or β3d subunit proteins and is likely to be an inhibitor or activator of calcium sensitive potassium channels containing human calcium sensitive potassium channel β3a, β3b, β3c, or β3d subunit proteins.

17. A method of identifying activators or inhibitors of calcium sensitive potassium channels containing human calcium sensitive potassium channel β3a, β3b, β3c, or β3d subunit proteins comprising:
(a) recombinantly expressing human calcium sensitive potassium channel β3a, β3b, β3c, or β3d subunit proteins or mutant human calcium sensitive potassium channel β3a, β3b, β3c, or β3d subunit proteins in a host cell so that the recombinantly expressed human calcium sensitive potassium channel β3a, β3b, β3c, or β3d subunit proteins form calcium sensitive potassium channels by forming heteromers with other calcium sensitive potassium channel subunit proteins;
(b) measuring the biological activity of the calcium sensitive potassium channels formed in step (a) in the presence and in the absence of a substance suspected of being an activator or an inhibitor of calcium sensitive potassium channels containing human calcium sensitive potassium channel β3a, β3b, β3c, or β3d subunit proteins;
where a change in the biological activity of the calcium sensitive potassium channels formed in step (a) in the presence as compared to the absence of the substance indicates that the substance is an activator or an inhibitor of calcium sensitive potassium channels containing human calcium sensitive potassium channel β3a, β3b, β3c, or β3d subunit proteins.

18. A method of identifying DNA sequences in the β3 gene that promote, enhance, or repress gene transcription comprising:
(a) constructing a promoter-reporter vector such that fragments of the promoter region of the β3 gene (SEQ.ID.NO.:20, nucleotides 1 to 17,436) precede the coding cDNA sequence of a reporter gene which encodes a reporter protein;
(b) transfecting the vector into cells and measuring the abundance of the reporter protein encoded by the vector;
(c) comparing the abundance of the reporter protein in the cells of step (b) to the abundance of the reporter protein in cells transfected with the vector without fragments of the promoter region of the β3 gene;
where fragments of the promoter region of the β3 gene which increase the abundance of the reporter protein in the absence of other promoter elements only in cells which endogenously express β3a, β3b, β3c, or β3d subunits are promoter elements; sequences which decrease the abundance of the reporter protein in the presence of an unrelated constitutive promoter element in cells which do not endogenously express β3a, β3b, β3c, or β3d subunits are repressor elements; and sequences which increase the abundance of the reporter protein in the presence of an unrelated constitutive promoter element in cells which endogenously express β3a, β3b, β3c, or β3d subunits are enhancer elements.

19. The method of claim 18 where the vector contains promoter or enhancer sequence elements which function independently of the fragments of the promoter region of the β3 gene.

20. The method of claim 18 where the abundance of the reporter protein is normalized with respect to the fraction of transfected cells.

21. A method of identifying DNA sequences in the β3 gene that promote, enhance, or repress gene transcription comprising:
(a) incubating radiolabeled fragments of double stranded DNA corresponding to sequences found in the promoter region of the β3 gene (SEQ.ID.NO.:20, nucleotides 1 to 17,436) with nuclear extracts from cells; and
(b) separating the incubation on a gel;
where fragments of double stranded DNA corresponding to sequences found in the promoter region of the β3 gene that migrate differently in a gel ('undergo a shift') after incubation with nuclear extracts from cells are DNA sequences which bind nuclear factors which promote, enhance or repress β3 gene expression.

22. The method of claim 21 where the fragments of double stranded DNA corresponding to sequences found in the promoter region of the β3 gene are identified by the method of claim 18.

23. The method of claim 21 where the cells express β3a, β3b, β3c, or β3d subunits.

24. The method of claim 21 where the cells do not express β3a, β3b, β3c, or β3d subunits.

25. A method of identifying nuclear factors involved in β3 gene transcription regulation comprising:
(a) incubating radiolabeled fragments of double stranded DNA corresponding to sequences found in the promoter region of the β3 gene (SEQ.ID.NO.:20, nucleotides 1 to 17,436) with cloned or purified transcription factors and separating the incubation on a gel;
where factors which bind β3 gene promoter sequence elements will induce a shift in the migration of the radiolabeled DNA fragments, and are involved in β3 gene transcription regulation.

26. The method of claim 25 where the fragments of double stranded DNA corresponding to sequences found in the promoter region of the β3 gene are identified by the methods of claim 18 or 21.

27. A method of identifying transcription factors involved in β3 gene transcription regulation comprising:
(a) incubating radiolabeled fragments of double stranded DNA corresponding to sequences found in the promoter region of the β3 gene (SEQ.ID.NO.:20, nucleotides 1 to 17,436) with nuclear extracts from cells and separating the incubation on a gel;
(b) adding an antibody that specifically recognizes a single transcription factor or a family of transcription factors to the incubation of step (a), followed by separating the incubation on a gel;
where a super-shift in mobility of the double stranded DNA in step (b) as compared to step (a) indicates that a transcription factor recognized by the antibody binds the double stranded DNA.

28. A method of identifying clones encoding nuclear factors involved in β3 gene transcription regulation by cloning comprising:
(a) screening an expression library with radiolabeled fragments of double stranded DNA corresponding to sequences found in the promoter region of the β3 gene (SEQ.ID.NO.:20, nucleotides 1 to 17,436)
(b) determining which clones of the library bind the radiolabeled fragments of double stranded DNA;
(c) amplifying and sequencing the clones of step (b).

29. The method of claim 28 where the fragments of double stranded DNA corresponding to sequences found in the promoter region of the β3 gene are identified by the methods of claim 18 or 21.

30. A method of identifying nuclear factors involved in β3 gene transcription regulation by cloning comprising:
(a) attaching fragments of double stranded DNA corresponding to sequences found in the promoter region of the β3 gene (SEQ.ID.NO.:20, nucleotides 1 to 17,436) to a stable matrix;
(b) incubating phage expressing cDNA encoded fusion proteins at their surface with the matrix;
(c) removing phage that do not bind to the matrix by washing;
(d) eluting phage bound to the matrix with excess fragments of double stranded DNA corresponding to sequences found in the promoter region of the β3 gene;
where the phage eluted in step (d) encode nuclear factors involved in β3 gene transcription regulation.

31. The method of claim 30 where the DNA corresponding to sequences found in the promoter region of the β3 gene are identified by the methods of claim 18 or 21.

32. The method of claim 30 where the phage eluted at step (d) are amplified and sequenced.

33. A method of identifying nuclear factors involved in β3 gene transcription regulation comprising:
(a) attaching fragments of double stranded DNA corresponding to sequences found in the promoter region of the β3 gene (SEQ.ID.NO.:20, nucleotides 1 to 17,436) to a stable matrix;
(b) incubating nuclear extracts from cells with the matrix;
(c) washing non-binding proteins from the nuclear extract from the matrix;
(d) eluting bound proteins from the matrix with excess double stranded DNA corresponding to sequences found in the promoter region of the β3 gene;
where the eluted proteins from step (d) are nuclear factors involved in β3 gene transcription regulation.

34. The method of claim 33 further comprising separating the eluted proteins from step (d) on a gel and staining the gel to test for purity of the eluted proteins.

35. The method of claim 34 further comprising sequencing the proteins that have been separated on the gel.

36. The method of claim 34 further comprising immunological analysis of the proteins that have been separated on the gel with antibodies directed towards known transcription factors to identify the eluted proteins by western blot or immunoprecipitation.

37. The method of claim 33 where the fragments of double stranded DNA corresponding to sequences found in the promoter region of the β3 gene are identified by the methods of claim 18 or 21.

38. A method of identifying nuclear factors involved in β3 gene transcription regulation by cloning comprising:
(a) constructing a yeast strain that contains a few to several copies of a fragment of double stranded DNA corresponding to sequences found in the promoter region of the β3 gene (SEQ.ID.NO.:20, nucleotides 1 to 17,436) preceding a cDNA encoding a reporter protein;
(b) constructing a cDNA library from cells in a vector that allows formation of fusion proteins encoded by the inserted cDNA and a transcription activation domain;
(c) transforming the library of (b) into the yeast strain of (a) and isolating colonies of yeast displaying expression of the reporter protein.

39. The method of claim 38 where the fragments of double stranded DNA corresponding to sequences found in the promoter region of the β3 gene are identified by the methods of claim 18 or 21.

40. The method of claim 38 further comprising purifying the vectors from the isolated colonies and sequencing the cDNA in the vectors.

41. A method of identifying substances that enhance or inhibit the rate of transcription of the β3 gene comprising:
(a) constructing a promoter-reporter vector such that fragments of the promoter region of the β3 gene (SEQ.ID.NO.:20, nucleotides 1 to 17,436) precede the coding cDNA sequence of a reporter gene which encodes a reporter protein;
(b) transfecting the vector into cells and measuring the abundance of the reporter protein encoded by the vector in the presence and absence of a compound;
where (1) if the presence of the compound decreases the abundance of the reporter protein, then the compound is a substance that inhibits the rate of transcription of the β3 gene; (2) if the presence of the compound increases the abundance of the reporter protein, then the compound is a substance that enhances the rate of transcription of the β3 gene.

42. The method of claim 41 further comprising a control in which the effect of the compound on the abundance of the reporter protein in control cells is measured, where the control cells are cells that are essentially the same as the cells of step (b) except that the control cells have been transfected with a vector that lacks fragments of the promoter region of the β3 gene.
